# NEUE EUROPÄISCHE PATENTSCHRIFT

(11) **EP 1 562 603 B3**
(45) Veröffentlichungstag dieser Patentschrift: **08.01.2014**
(45) Hinweis auf die Patenterteilung: 06.05.2009
(21) Anmeldenummer: 03778312.3
(22) Anmeldetag: 11.11.2003
(51) Int. Cl.: A61K 31/538, A61P 11/00, A61K 31/535

(54) **NEUE ARZNEIMITTEL ZUR BEHANDLUNG VON CHRONISCH OBSTRUKTIVER LUNGENERKRANKUNG**
NOVEL MEDICAMENTS FOR THE TREATMENT OF CHRONIC OBSTRUCTIVE PULMONARY DISEASES
NOUVEAUX MEDICAMENTS DESTINES AU TRAITEMENT DE LA BRONCHOPNEUMOPATHIE CHRONIQUE OBSTRUCTIVE

(30) Priorität: 15.11.2002 DE 10253282
(43) Veröffentlichungstag der Anmeldung: 17.08.2005
(60) Teilanmeldung: 08169872.2
(73) Patentinhaber: Boehringer Ingelheim Pharma GmbH & Co. KG, 55216 Ingelheim am Rhein (DE)
(72) Erfinder: BOUYSSOU, Thierry, 88447 Warthausen (DE); BUETTNER, Frank, 88448 Attenweiler (DE); KONETZKI, Ingo, 88447 Warthausen (DE); PESTEL, Sabine, 88448 Attenweiler (DE); SCHNAPP, Andreas, 88400 Biberach (DE); SCHOLLENBERGER, Hermann, 55218 Ingelheim (DE); SCHROMM, Kurt, 55218 Ingelheim (DE); HEINE, Claudia, 88400 Biberach (DE); RUDOLF, Klaus, 88447 Warthausen (DE); LUSTENBERGER, Philipp, 88447 Warthausen (DE); HOENKE, Christoph, 55218 Ingelheim (DE)
(74) Vertreter: HOFFMANN EITLE
(86) Internationale Anmeldenummer: PCT/EP2003/012565
(87) Internationale Veröffentlichungsnummer: WO 2004/045618

(56) Entgegenhaltungen:
- EP-A- 0 073 505
- EP-A- 0 321 864
- US-A- 4 460 581

## Beschreibung

Die vorliegende Erfindung betrifft die Verwendung von Verbindungen, die eine spezielle Ausführungsform der Verbindungen der allgemeinen Formel 1 sind 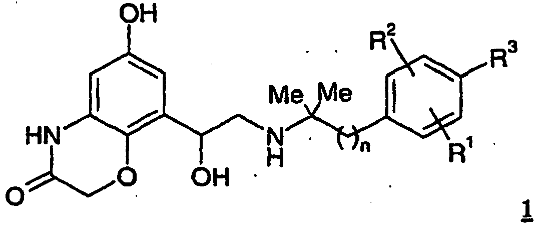 worin die Reste R¹, R² und R³ die in den Ansprüchen und in der Beschreibung genannten Bedeutungen haben können, zur Herstellung eines Arzneimittels zur Behandlung von COPD (chronisch obstruktive Lungenerkrankung = chronic obstructive pulmonary disease), sowie Verbindungen, die eine spezielle Ausführungsform der neuer Verbindungen der allgemeinen Formel 1 sind und Verfahren zu deren Herstellung.

### Hintergrund der Erfindung

Betamimetika (β-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.
Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Es ist daher Aufgabe der vorliegenden Efindung, Betamimetika bereitzustellen, die einerseits bei der Therapie der COPD einen therapeutischen Nutzen entfalten und darüberhinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von COPD einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind.

### Detaillierte Beschreibung der Efindung

Überraschenderweise wurde gefunden, daß die vorstehend genannten Aufgaben durch Verbindungen der allgemeinen Formel 1 gelöst werden, insbesondere durch die Verbindungen der Ansprüche 1-7, die eine spezielle Ausführungsform der Verbindungen der allgemeinen Formel 1 sind.

Folgende verbindungen bei denen n 1 oder 2 sein kann und deren verwendung sind nicht als teil der erfindung anzusehen und als referenz zu betrachten.

Dementsprechend betrifft die vorliegende Erfindung die Verwendung von Verbindungen der allgemeinen Formel 1, insbesondere von den Verbindungen der Ansprüche 1-7, die eine spezielle Ausführungsform der Verbindungen der allgemeinen Formel 1 sind, 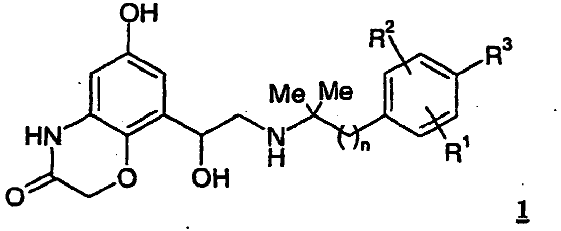 worin
- n: 1 oder 2,
- R¹: Wasserstoff, C₁-C₄-Alkyl, Halogen, OH oder -O-C₁-C₄-Alkyl;
- R²: Wasserstoff, C₁-C₄-Alkyl, Halogen, OH oder -O-C₁-C₄-Alkyl;
- R³: Wasserstoff, C₁-C₄-Alkyl, OH, Halogen, -O-C₁-C₄-Alkyl, -O-C₁-C₄-Alkylen-COOH oder -O-C₁-C₄-Alkylen-CO-O-C₁-C₄-Alkyl,
bedeuten, zur Herstellung eines Arzneimittels zur Behandlung von COPD.

Bevorzugt ist die Verwendung von Verbindungen der allgemeinen Formel 1, worin
- n: 1 oder 2,
- R¹: Wasserstoff, Halogen oder C₁-C₄-Alkyl;
- R²: Wasserstoff, Halogen oder C₁-C₄-Alkyl;
- R³: Wasserstoff, C₁-C₄-Alkyl, OH, Halogen, -O-C₁-C₄-Alkyl, -O-C₁-C₄-Alkylen-COOH oder -O-C₁-C₄-Alkylen-CO-O-C₁-C₄-Alkyl,
bedeuten, zur Herstellung eines Arzneimittels zur Behandlung von COPD.

Bevorzugt ist die Verwendung von Verbindungen der allgemeinen Formel 1, worin
- n: 1 oder 2;
- R¹: Wasserstoff, Fluor, Chlor oder Methyl;
- R²: Wasserstoff, Fluor, Chlor oder Methyl;
- R³: Wasserstoff, C₁-C₄-Alkyl, OH, Fluor, Chlor, Brom, -O-C₁-C₄-Alkyl, -O-C₁-C₄-Alkylen-COOH, -O-C1-C4-Alkylen-CO-O-C₁-C₄-Alkyl, bedeuten,
zur Herstellung eines Arzneimittels zur Behandlung von COPD.

Besonders bevorzugt ist die Verwendung von Verbindungen der allgemeinen Formel 1, worin
- n: 1 oder 2,
- R¹: Wasserstoff, Methyl oder Ethyl ;
- R²: Wasserstoff, Methyl oder Ethyl ;
- R³: Wasserstoff, Methyl, Ethyl, OH, Methoxy, Ethoxy, -O-CH₂-COOH, -O-CH₂-CO-O-Methyl oder -O-CH₂-COOEthyl;
bedeuten, zur Herstellung eines Arzneimittels zur Behandlung von COPD.

Besonders bevorzugt ist die Verwendung von Verbindungen der allgemeinen Formel 1, worin
- n: 1 oder 2,
- R¹: Wasserstoff oder Methyl;
- R²: Wasserstoff oder Methyl;
- R³: Wasserstoff, Methyl, OH, Methoxy, -O-CH₂-COOH oder -O-CH₂-COOEthyl;
bedeuten, zur Herstellung eines Arzneimittels zur Behandlung von COPD.

Erfindungsgemäß von besonderer Bedeutung ist ferner die Verwendung von Verbindungen der allgemeinen Formel **1**, worin
- n: 1 oder 2,
- R¹: Wasserstoff oder Methyl;
- R²: Wasserstoff oder Methyl;
- R³: Wasserstoff, OH, Methoxy oder -O-CH₂-COOH;
bedeuten, zur Herstellung eines Arzneimittels zur Behandlung von COPD.

Ein bevorzugter Aspekt der vorliegenden Erfindung zielt ferner auf die Verwendung von Verbindungen der allgemeinen Formel **1** in denen n = 1 ist und die Reste R¹ , R² und R³ die vorstehend genannten Bedeutungen haben können, zur Herstellung eines Arzneimittels zur Behandlung von COPD.

Ein weiterer bevorzugter Aspekt der vorliegenden Erfindung betrifft die Verwendung von Verbindungen der allgemeinen Formel **1** in denen n = 1 oder 2 , R³ ein Rest ausgewählt aus der Gruppe bestehend aus Wasserstoff, OH, -O-C₁-C₄-Alkyl und -O-C₁-C₄-Alkylen-COOH und in denen die Reste R¹ und R² die vorstehend genannten Bedeutungen haben können, zur Herstellung eines Arzneimittels zur Behandlung von COPD.

Ein weiterer bevorzugter Aspekt der vorliegenden Erfindung betrifft die Verwendung von Verbindungen der allgemeinen Formel **1** in denen n = 2 , R¹ und R² Wasserstoff und der Rest R³ die vorstehend genannten Bedeutungen haben kann, zur Herstellung eines Arzneimittels zur Behandlung von COPD.

In den Verbindungen der Formel **1** können die Reste R¹ und R², sofern sie nicht Wasserstoff bedeuten, jeweils ortho oder meta bezüglich der Verknüpfung zur benzylischen "-CH₂"-Gruppe angeordnet sein. Wenn keiner der Reste R¹ und R² Wasserstoff bedeutet, ist die erfindungsgemäße Verwendung derjenigen Verbindungen der Formel **1** bevorzugt, in denen beide Reste R¹ und R² entweder ortho oder beide Reste R¹ und R² meta konfiguriert sind, wobei der Verwendung derjenigen Verbindungen, in denen beide Reste R¹ und R² ortho-konfiguruert sind, besondere Bedeutung zukommt. In den Verbindungen der Formel **1** in denen einer der Reste R¹ und R² nicht Wasserstoff bedeutet, kann dieser ortho oder meta bezüglich der Verknüpfung zur benzylischen "-CH₂"-Gruppe angeordnet sein. In diesem Fall ist insbesondere die erfindungsgemäße Verwendung derjenigen Verbindungen der Formel **1** bevorzugt, in denen der Rest R¹ oder R², der nicht Wasserstoff bedeutet, ortho-konfiguriert ist.

Ein weiterer Aspekt der vorliegenden Erfindung betrifft die vorstehend genannte Verwendung der Verbindungen der Formel **1** in Form der einzelnen optischen Isomeren, Mischungen der einzelnen Enantiomeren oder Racemate. Besonders bevorzugt ist hierbei die vorstehend genannte Verwendung der Verbindungen der Formel **1** in Form der enantiomerenreinen Verbindungen, wobei die Verwendung der R-Enantiomere der Verbindungen der Formel **1** erfindungsgemäß von herausragender Bedeutung ist.

Ein weiterer Aspekt der vorliegenden Erfindung betrifft die vorstehend genannte Verwendung der Verbindungen der Formel **1** in Form der Säureadditionssalze mit pharmakologisch unbedenklichen Säuren sowie gegebenenfalls in Form der Solvate und/oder Hydrate.

Die vorliegende Erfindung betrifft ferner die Verwendung der vorstehend genannten Verbindungen der allgemeinen Formel **1** zur Herstellung eines Arzneimittels zur einmal täglichen Behandlung von COPD.

Ferner betrifft die vorliegende Erfindung ein Verfahren zur Behandlung von COPD, dadurch gekennzeichnet, daß eine oder mehrere der vorstehend genannten Verbindungen der allgemeinen Formel **1** in therapeutisch wirksamen Mengen appliziert werden. Die vorliegende Erfindung betrifft ferner Verfahren zur Behandlung von COPD, dadurch gekennzeichnet, daß eine oder mehrere der vorstehend genannten Verbindungen der allgemeinen Formel **1** in therapeutisch wirksamen Mengen einmal täglich appliziert werden.

Die Verbindungen der allgemeinen Formel **1** sind teilweise im Stand der Technik bekannt. Hierzu sei auf die Offenbarung der US 4460581 verwiesen. Teilweise sind die Verbindungen der allgemeinen Formel **1** allerdings noch nicht im Stand der Technik offenbart. Ein weiterer Aspekt der vorliegenden Erfindung betrifft diese neuen Verbindungen der Formel **1** als solche.

EP0073505 offenbart in allgemeiner weise verbindungen die Formel **1** umfassen, die für die behandlung von U.A. asthma und bronchitis geeignet sind.

Dementsprechend betrifft die vorliegende Erfindung ferner Verbindungen der allgemeinen Formel **1**, insbesondere die Verbindungen der Ansprüche 1-7, die eine spezielle Ausführungsform der Verbindungen der allgemeinen Formel 1 sind, 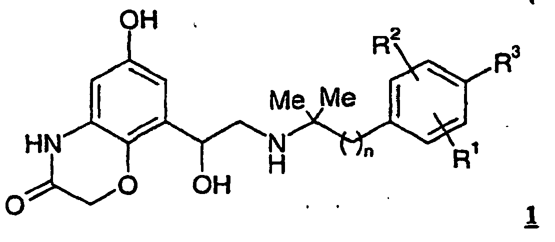 worin
- n: 1;
- R¹: Wasserstoff, Halogen, C₁-C₄-Alkyl oder -O-C₁-C₄-Alkyl;
- R²: Wasserstoff, Halogen, C₁-C₄-Alkyl oder -O-C₁-C₄-Alkyl;
- R³: C₁-C₄-Alkyl, OH, Halogen, -O-C₁-C₄-Alkyl, -O-C₁-C₄-Alkylen-COOH, -O-C₁-C₄-Alkylen-CO-O-C₁-C₄-Alkyl.
bedeuten, mit der Maßgabe, daß wenn R¹ und R² jeweils ortho-Methyl bedeuten, R³ nicht gleichzeitig OH sein kann.

Bevorzugt sind Verbindungen der allgemeinen Formel 1, worin
- n: 1;
- R¹: Wasserstoff, Fluor, Chlor, Methyl oder Methoxy;
- R²: Wasserstoff, Fluor, Chlor, Methyl oder Methoxy;
- R³: C₁-C₄-Alkyl, OH, Fluor, Chlor, Brom, -O-C₁-C₄-Alkyl, -O-C₁-C₄-Alkylen-COOH, -O-C₁-C₄-Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

Bevorzugt sind Verbindungen der allgemeinen Formel **1** worin
- n: 1;
- R¹: Wasserstoff oder C₁-C₄-Alkyl;
- R²: Wasserstoff oder C₁-C₄-Alkyl;
- R³: C₁-C₄-Alkyl, OH, -O-C₁-C₄-Alkyl, -O-C₁-C₄-Alkylen-COOH oder -O-C₁-C₄- Alkylen-CO-O-C₁-C₄-Alkyl,
bedeuten, mit der Maßgabe, daß wenn R¹ und R² jeweils ortho-Methyl bedeuten, R³ nicht gleichzeitig OH sein kann.

Bevorzugt sind die Verbindungen der allgemeinen Formel 1, worin
- n: 1;
- R¹: Wasserstoff, Methyl oder Ethyl;
- R²: Wasserstoff, Methyl oder Ethyl;
- R³: Methyl, Ethyl, OH, Methoxy, Ethoxy, -O-CH₂-COOH, -O-CH₂-COOMethyl oder -O-CH₂-COOEthyl,
bedeuten, mit der Maßgabe, daß wenn R¹ und R² jeweils ortho-Methyl bedeuten, R³ nicht gleichzeitig OH sein kann.

Bevorzugt sind ferner die Verbindungen der allgemeinen Formel **1**, worin
- n: 1;
- R¹: Wasserstoff oder Methyl;
- R²: Wasserstoff oder Methyl;
- R³: Methyl, OH, Methoxy, -O-CH₂-COOH oder -O-CH₂-COOEthyl,
bedeuten, mit der Maßgabe, daß wenn R¹ und R² jeweils ortho-Methyl bedeuten, R³ nicht gleichzeitig OH sein kann.

Erfindungsgemäß bevorzugt sind ferner Verbindungen der allgemeinen Formel **1**, worin
- R³: Methoxy, Ethoxy, -O-CH₂-COOH, -O-CH₂-COOMethyl oder -O-CH₂-COOEthyl,
bedeuten und R¹, R² und n die vorstehend genannten Bedeutungen haben können.

Die vorliegende Erfindung betrifft ferner Verbindungen der allgemeinen Formel **1**. worin
- n: 1;
- R¹: Halogen, C₁-C₄-Alkyl oder -O-C₁-C₄-Alkyl;
- R²: Halogen, C₁-C₄-Alkyl oder -O-C₁-C₄-Alkyl;
- R³: Halogen, C₁-C₄-Alkyl oder -O-C₁-C₄-Alkyl, bedeuten.

Die vorliegende Erfindung betrifft ferner Verbindungen der allgemeinen Formel 1, worin
- n: 1;
- R¹: Fluor, Chlor, Methyl oder Methoxy;
- R²: Fluor, Chlor, Methyl oder Methoxy ;
- R³: Fluor, Chlor, Methyl oder Methoxy bedeuten.

Ein weiterer bevorzugter Aspekt der vorliegenden Erfindung betrifft die Verbindungen der allgemeinen Formel 1 worin
- n: 1;
- R¹: Wasserstoff;
- R²: Wasserstoff, Fluor, Chlor oder Methyl;
- R³: Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COOMethyl, -O-CH₂-CH₂-COOEthyl, -O-CH₂-CH₂-CH₂-COOMethyl, -O-CH₂-CH₂-CH₂-COOEthyl, bedeuten.

Besonders bevorzugt sind ferner Verbindungen der allgemeinen Formel **1** worin
- n: 1;
- R¹: Wasserstoff;
- R²: Wasserstoff, Fluor, Chlor oder Methyl;
- R³: OH, Fluor, Chlor, Methyl, Methoxy, Ethoxy oder -O-CH₂-COOH, bedeuten.

Ferner sind erfindungsgemäß besonders bevorzugt Verbindungen der allgemeinen Formel **1** worin
- n: 1;
- R¹: Wasserstoff;
- R²: Halogen, C₁-C₄-Alkyl oder -O-C₁-C₄-Alkyl, bevorzugt Fluor, Chlor, Methoxy oder Methyl;
- R³: Halogen, C₁-C₄-Alkyl oder -O-C₁-C₄-Alkyl, bevorzugt Fluor, Chlor, Methoxy oder Methyl, bedeuten.

Ein weiterer bevorzugter Aspekt der vorliegenden Erfindung betrifft die Verbindungen der allgemeinen Formel **1** in denen n = 1 , R¹ und R² Wasserstoff und der Rest R³ die vorstehend genannten Bedeutungen haben kann.

Ein weiterer bevorzugter Aspekt der vorliegenden Erfindung betrifft die Verbindungen der allgemeinen Formel **1**, worin
- n: 1;
- R¹ und R²: Wasserstoff;
- R³: Methyl, Ethyl, iso-Propyl, tert.-Butyl, OH, Fluor, Chlor, Brom, Methoxy, Ethoxy, -O-CH₂-COOH, -O-CH₂-CH₂-COOH, -O-CH₂-CH₂-CH₂-COOH, -O-CH₂-COOMethyl, -O-CH₂-COOEthyl, -O-CH₂-CH₂-COO-Methyl, -O-CH₂-CH₂-COOEthyl, -O-CH₂-CH₂-CH₂-COOMethyl, -O-CH₂-CH₂-CH₂-COOEthyl, bedeuten.

Besonders bevorzugt sind Verbindungen der allgemeinen Formel **1**, worin
- n: 1;
- R¹ und R²: Wasserstoff;
- R³: OH, Fluor, Chlor, Methoxy, Ethoxy,-O-CH₂-COOH, bevorzugt OH, Fluor, Chlor, Ethoxy oder Methoxy, bedeuten.

Besonders bevorzugt sind Verbindungen der allgemeinen Formel **1**, worin
- n: 1;
- R¹ und R²: Wasserstoff;
- R³: Fluor, Chlor, Methoxy oder Ethoxy bedeuten.

Die vorliegende Erfindung betrifft ferner Verbindungen der allgemeinen Formel **1**, worin
- n: 1;
- R¹: Wasserstoff, Halogen, C₁-C₄-Alkyl oder -O-C₁-C₄-Alkyl;
- R²: Wasserstoff, Halogen, C₁-C₄-Alkyl oder -O-C₁-C₄-Alkyl;
- R³: Wasserstoff, bedeuten.

Bevorzugt sind dabei Verbindungen der allgemeinen Formel **1**, worin
- n: 1;
- R¹: Wasserstoff, Fluor, Chlor, Methyl oder Methoxy;
- R²: Wasserstoff, Fluor, Chlor, Methyl oder Methoxy;
- R³: Wasserstoff, bedeuten.

Die vorliegende Efindung betrifft ferner Verbindungen der allgemeinen Formel **1**. worin
- n: 1;
- R¹: Fluor, Chlor, Methyl oder Methoxy;
- R²: Fluor, Chlor, Methyl oder Methoxy;
- R³: Wasserstoff, bedeuten.

In den Verbindungen der Formel **1** können die Reste R¹ und R², sofern sie nicht Wasserstoff bedeuten, jeweils ortho oder meta bezüglich der Verknüpfung zur benzylischen "-CH₂"-Gruppe angeordnet sein. Wenn keiner der Reste R¹ und R² Wasserstoff bedeutet, sind diejenigen Verbindungen der Formel **1** bevorzugt, in denen beide Reste R¹ und R² entweder ortho oder beide Reste R¹ und R² meta konfiguriert sind, wobei Verbindungen, in denen beide Reste R¹ und R² ortho-konfiguriert sind, besondere Bedeutung zukommt.
In den Verbindungen der Formel **1** in denen einer der Reste R¹ und R² nicht Wasserstoff bedeutet, kann dieser ortho oder meta bezüglich der Verknüpfungzur benzylischen "-CH₂"-Gruppe angeordnet sein. In diesem Fall sind insbesondere diejenigen Verbindungen der Formel **1** bevorzugt, in denen der Rest R¹ oder R², der nicht Wasserstoff bedeutet, ortho-konfiguruert ist.

Besonders bevorzugt sind ferner Verbindungen der allgemeinen Formel **1**, die ausgewählt sind aus der Gruppe bestehend aus
- 6-Hydroxy-8-{1-hydroxy-2-[2-(4-methoxy-phenyl)-1,1-dimethyl-ethylamino]-ethyl}-4H-benzo[1,4]oxazin-3-on;
- 8-{2-[2-(2,4-Difluor-phenyl)-1,1-dimethyl-ethylamino]-1-hydroxy-ethyl}-6-hydroxy-4H-benzo[1,4]oxazin-3-on;
- 8-{2-[2-(3,5-Difluor-phenyl)-1,1-dimethyl-ethylamino]-1-hydroxy-ethyl}-6-hydroxy-4H-benzo[1,4]oxazin-3-on;
- 8-{2-[2-(4-Ethoxy-phenyl)-1,1-dimethyl-ethylamino]-1-hydroxy-ethyl}-6-hydroxy-4H-benzo[1,4]oxazin-3-on;
- 8-{2-[2-(4-Fluor-phenyl)-1,1-dimethyl-ethylamino]-1-hydroxy-ethyl}-6-hydroxy-4H-benzo[1,4]oxazin-3-on;

Ein weiterer Aspekt der vorliegenden Erfindung betrifft die vorstehend genannten neuen Verbindungen der Formel **1** in Form der einzelnen optischen isomeren, Mischungen der einzelnen Enantiomeren oder Racemate. Besonders bevorzugt sind dabei Verbindungen der Formel **1** in Form der enantiomerenreinen Verbindungen, wobei die R-Enantiomere der Verbindungen der Formel **1** erfindungsgemäß von herausragender Bedeutung sind. Verfahren zur Auftrennung von Racematen in die jeweiligen Enantiomere sind im Stand der Technik bekannt und können zur Darstellung der enantiomerenreinen R- bzw. S-Enantiomere der Verbindungen der Formel **1** in analoger Art und Weise zur Anwendung gelangen.

Ein weiterer Aspekt der vorliegenden Erfindung betrifft die vorstehend genannten Verbindungen der Formel **1** in Form ihrer Säureadditionssalze mit pharmakologisch unbedenklichen Säuren sowie gegebenenfalls in Form der Solvate und/oder Hydrate.

Ein weiterer Aspekt der vorliegenden Erfindung betrifft die vorstehend genannten neuen Verbindungen der Formel **1** zur Verwendung als Arzneimittel. Ferner betrifft die vorliegende Erfindung die Verwendung der vorstehend genannten neuen Verbindungen der Formel **1** zur Herstellung eines Arzneimittles zur Behandlung von COPD. Ferner betrifft die vorliegende Erfindung die Verwendung der vorstehend genannten neuen Verbindungen der Formel **1** zur Herstellung eines Arzneimittles zur einmal täglichen Behandlung von COPD.

Ferner betrifft die vorliegende Erfindung ein Verfahren zur Behandlung von COPD, dadurch gekennzeichnet, daß eine oder mehrere der vorstehend genannten neuen Verbindungen der allgemeinen Formel **1** in therapeutisch wirksamen Mengen appliziert werden. Die vorliegende Erfindung betrifft ferner Verfahren zur Behandlung von COPD, dadurch gekennzeichnet, daß eine oder mehrere der vorstehend genannten neuen Verbindungen der allgemeinen Formel 1 in therapeutisch wirksamen Mengen einmal täglich appliziert werden.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Zur erfindungsgemäßen Verwendung können die Verbindungen der allgemeinen Formel **1** gegebenenfalls in Form der einzelnen optischen isomeren, Mischungen der einzelnen Enantiomeren oder Racemate zur Anwendung gelangen. Werden die Verbindungen in enantiomerenreiner Form eingesetzt, werden bevorzugt die R-Enantiomere verwendet.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, n-Propylen oder n-Butylen.

Als Alkyloxygruppen (oder auch -O-Alkylgruppen) werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO-verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4460581 bekannt.

Die nachstehend beschriebenen Beispiele dienen der weitergehenden Illustration von aus dem Stand der Technik bekannten Verbindungen.

### Beispiel 1: 6-Hydroxy-8-{1-hydroxy-2-[2-(4-hydroxy-2,6-dimethyl-phenyl)-1,1-dimethylethylamino]-ethyl}-4H-benzo [1,4]oxazin-3-on

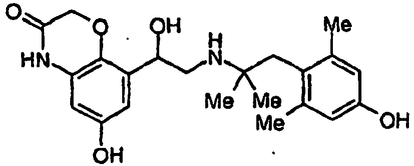

Die Verbindung ist aus der US 4460581 bekannt.

### Beispiel 2: 8-{2-(1,1-Dimethyl-3-phenyl-propylamino]-1-hydroxy-ethyl}-6-hydroxy-4H-benzo[1,4]oxazin-3-on

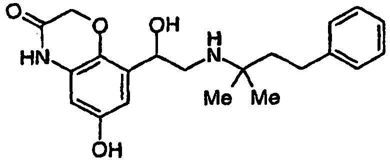

Die Verbindung ist aus der US 4460581 bekannt.

Die nachstehend beschriebenen Synthesebeispiele dienen der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel 3: 6-Hydroxy-8-{1-hydroxy-2-[2-(4-methoxy-phenyl)-1,1-dimethyl-ethylamino]-ethyl}-4H-benzo[1,4]oxazin-30-on

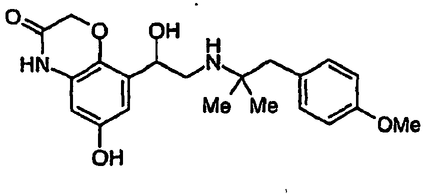

### a) 8-{2-[1,1-Dimethyl-2-(4-methoxy-phenyl)-ethylamino]-1-hydroxy-ethyl}-6-benzyloxy-4H-benzo[1,4]oxazin-3-on

Zu einer Lösung von 3.6 g 1,1-Dimethyl-2-(4-methoxyphenyl)-ethylamin in 100 mL Ethanol werden bei 70°C 7.5 g (6-Benzyloxy-4H-benzo[1,4]oxazin-3-on)-glyoxalhydrat gegeben und man läßt 15 Minuten rühren. Anschließend werden innerhalb von 30 Minuten bei 10 bis 20°C 1 g Natriumborhydrid zugesetzt. Es wird eine Stunde gerührt, mit 10 mL Aceton versetzt und über weitere 30 Minuten gerührt. Die Reaktionsmischung wird mit 150 mL Ethylacetat verdünnt, mit Wasser gewaschen, mit Natriumsulfat getrocknet und eingeengt. Der Rückstand wird in 50 mL Methanol und 100 mL Ethylacetat gelöst und mit konz. Salzsäure sauer gestellt. Nach Zugabe von 100 mL Diethylether fällt das Produkt aus. Die Kristalle werden abfiltriert, gewaschen und in 50 mL Ethanol umkristallisiert. Ausbeute: 7 g (68%; Hydrochlorid); Schmp. = 232-234°C.

### b) 8-{2-[1,1-Dimethyl-2-(4-methoxy-phenyl)-ethylamino]-1-hydroxy-ethyl}-6-hydroxy-4H-benzo[1,4]oxazin-3-on

6.8 g der vorstehend erhaltenen Benzylverbindung werden in 125 mL Methanol unter Zusatz von 1 g Palladium auf Kohle (5%ig) bei Raumtemperatur und Normaldruck hydriert. Der Katalysator wird abfiltriert und das Filtrat vom Lösungsmittel befreit. Nach Umkristallisation des Rückstandes in 50 mL Aceton und etwas Wasser wird ein Feststoff erhalten, der abfiltriert und gewaschen wird.
Ausbeute: 5.0 g (89 %; Hydrochlorid); Schmp. =155-160°C.

Die (R)- und (S)-Enantiomere von Beispiel 3 können aus dem Racemat beispielsweise mittels chiraler HPLC erhalten werden (z.B. Säule: Chirobiotic T, 250 x 22.1 mm von der Firma Astec). Als mobile Phase kann Methanol mit 0.05 % Triethylamin und 0.05% Essigsäure verwendet werden. Kieselgel mit einer Korngröße von 5 µm, an das kovalent das Glykoprotein Teicoplanin gebunden ist, kann als Säulenmaterial zum Einsatz gelangen. Retentionszeit (R-Enantiomer) = 40.1 min, Retentionszeit (S-Enatiomer) = 45.9 min. Die beiden Enantiomere werden nach dieser Methode in Form der freien Basen erhalten. Erfindungsgemäß von herausragender Bedeutung ist das R-Enantiomer des Beispiels 3.

### Vergleichsbiespiel 1: 6-Hydroxy-8{1-hydroxy-2-[2-(4-phenoxy-essigsäureethylester)-1,1-dimethylethylamino]-ethyl}-4H-benzo [1,4]oxazin-3-on

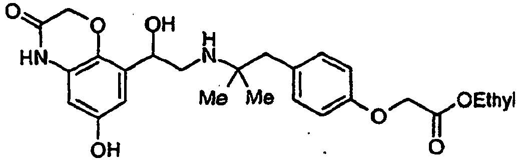

### a) 8-{2-[1,1-Dimethyl-2-(4-phenoxy-essigsäureethylester)-ethylamino]-1-hydroxy-ethyl}-6-benzyloxy-4H-benzo[1,4] oxazin-3-on

In Analogie zur unter Beispiel 3a) beschriebenen Vorgehensweise wird aus 15 g (6-Benzyloxy-4H-benzo[1,4] oxazin-3-on)-glyoxalhydrat und 11.8 g 1,1-Dimethyl-2-(4-phenoxy-essigsäureethylester)-ethylamin Hydrochlorid die Titelverbindung erhalten.
Ausbeute: 16.5 g (69%, Hydrochlorid); Schmp. = 212-214°C.

### b) 8-{2-[1,1-Dimethyl-2-(4-phenoxy-essigsäureethylester)-ethylamino]-1-hydroxy-ethyl}-6-hydroxy-4H-benzo[1,4] oxazin-3-on

8 g des vorstehend erhaltenen Benzylalkohols werden in 100 mL Ethanol, 100 mL Methanol und 10 mL Wasser gelöst und in Gegenwart von 1 g Palladium auf Kohle (5%ig) hydriert. Nach Aufnahme der theoretisch berechneten Menge Wasserstoff wird der Katalysator abfiltriert und das Filtrat eingeengt. Das beim Abdestillieren der Lösungsmittel auskristallisierende Produkt wird abgesaugt und gewaschen.
Ausbeute: 5.5 g (81%; Hydrochlorid); Schmp. = 137-140°C.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 2: 6-Hydroxy-8-{1-hydroxy-2-[2-(4-phenoxy-essigsäure)-1,1-dimethylethylamino]-ethyl}-4H-benzo[1,4] oxazin-3-on

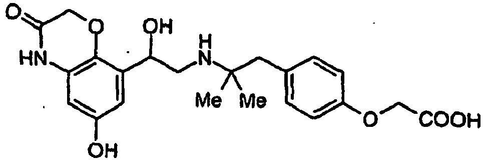

11 g 8-{2-[1,1-Dimethyl-2-(4-phenoxy-essigsäureethylester)-ethylamino]-1-hydroxyethyl}-6-benzyloxy-4H-benzo[1,4] oxazin-3-on hydrochlorid (Beispiel 4a) werden in 125 mL Methanol gelöst und in Gegenwart von 1 g Palladium auf Kohle (5%ig) hydriert. Nach Aufnahme der theoretisch berechneten Menge an Wasserstoff wird der Katalysator abfiltriert. Zum Filtrat werden 2.6 g Natriumhydroxid gelöst in 20 mL Wasser gegeben. Man läßt 30 Minuten refluxieren, destilliert das Methanol ab und versetzt mit 10 mL Wasser, 20 mL n-Butanol und 3.9 mL Essigsäure. Der ausfallende Feststoff wird abgesaugt und mit Diethylether gewaschen.
Ausbeute: 7 g (87%). Durch Umkristallisation aus 0.5 molarer Salzsäure wird das Hydrochlorid erhalten. Schmp. = 152°C.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 3: 8-{2-[1,1-Dimethyl-2-(2,4,6-trimethylphenyl)-ethylamino]1-1-hydroxy-ethyl)-6-hydroxy-4H-benzo[1,4] oxazin-3-on

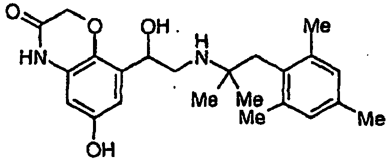

### a) 1-(6-Benzyloxy-4H-benzo[1,4]oxazin-3-on)-2-[1,1-dimethyl-2-(2,4,6-trimethylphenyl)-ethylimino]-ethanon

7.2 g (6-Benzyioxy-4H-benzo[1,4]oxazin-3-on)-glyoxalhydrat und 3.6 g 1,1-Dimethyl-2-(2,4,6-trimethylphenyl)-ethylamin werden eine Stunde in 100 mL Ethanol auf 70°C erwärmt. Nach dem Abkühlen werden die ausgefallenen Kristalle abfiltriert und mit Ethanol und Diethylether gewaschen. Ausbeute: 8.6 g (94%); Schmp. = 175°C.

### b) 8-{2-[1,1-Dimethyl-2-(2,4.6-trimethylphenyl)-ethylamino]-1-hydroxy-ethyl}-6-benzyloxy-4H-benzo[1,4]oxazin-3-on

8.6 g der nach der Vorschrift 6a) erhaltenen Schiffschen Base werden in 100 mL Ethanol und 20 mL THF gelöst, innerhalb von 30 min bei 10-20°C mit 0.7 g Natriumborhydrid versetzt und eine Stunde gerührt. Nach Zugabe von 10 mL Aceton wird 30 Minuten nachgerührt und dann mit Ethylacetat und Wasser verdünnt. Das beim Ansäuern mit konz. Salzsäure auskristallisierende Produkt wird abfiltriert und gewaschen.
Ausbeute: 7.4 g (80%, Hydrochlorid); Schmp. = 235°C (Zersetzung).

### c) 8-{2-[1,1-Dimethyl-2-(2,4,6-trimethylphenyl)-ethylamino]-1-hydroxy-ethyl}-6-hydroxy-4H-benzo[1,4]oxazin-3-on

7.4 g der unter Stufe b) erhaltenen Benzylverbindung werden in 125 mL Methanol unter Zusatz von 1 g Palladium auf Kohle (5%ig) bei Raumtemperatur und Normaldruck hydriert. Anschließend wird der Katalysator abfiltriert und das Filtrat eingeengt. Das bei Zugabe von Aceton auskristallisierende Produkt wird abgesaugt und mit Aceton und Diethylether gewaschen. Ausbeute: 5 g (78%, Hydrochlorid); Schmp. 160°C (Zersetzung).

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 4: 6-Hydroxy-8-{1-hydroxy-2-[2-(4-hydroxy-phenyl)-1,1-dimethyl-ethylamino]-ethyl}-4H-benzo[1,4]oxazin-3-on

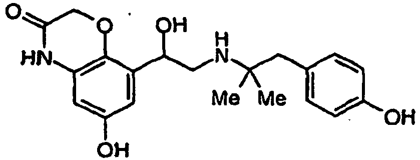

### a) 8-{2-[1,1-Dimethyl-2-(4-hydroxy-phenyl)-ethylaminol-1-hydroxy-ethyl}-6-benzyloxy-4H-benzo[1,4]oxazin-3-on

Die Titelverbindung wird aus 10 g (6-Benzyloxy-4H-benzo[1,4]oxazin-3-on)-glyoxalhydrat und 4.6 g 1,1-Dimethyl-2-(4-hydroxy-phenyl)-ethylamin analog zur Vorschrift für Beispiel 3a) hergestellt.
Ausbeute: 9.0 g (64%, Hydrochlorid); Schmp. = 255-258°C.

### b) 8-{2-[1,1-Dimethyl-2-(4-hydroxy-phenyl)-ethylamino]-1-hydroxy-ethyl}-6-hydroxy-4H-benzo[1,4]oxazin-3-on

5.7 g des vorstehend erhaltenen Kupplungsproduktes werden in Gegenwart von 0.6 g Palladium auf Kohle (5%ig) in 100 mL Methanol hydriert. Nach Aufnahme der theoretisch berechneten Menge an Wasserstoff wird der Katalysator abfiltriert und das Filtrat vom Lösungsmittel befreit. Der Rückstand wird unter Erwärmung in Ethanol gelöst und dann mit Diethylether versetzt. Das ausfallende Produkt wird abgesaugt und einmal in Wasser umkristallisiert. Ausbeute: 3.6 g (72%, Hydrochlorid); Schmp. = 159-162°C.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 5: 6-Hydroxy-8-{1-hydroxy-2-[2-(4-isopropyl-phenyl)-1,1dimethyl-ethylamino]-ethyl}-4H-benzo[1,4]oxazin-3-on

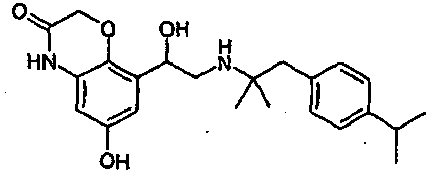

### a) 1-(4-Isopropyl-phenyl)-2-methyl-propan-2-ol

Die Umsetzung einer Grignardverbindung, hergestellt aus 20 g (119 mmol) 4-Isopropylbenzylchlorid, mit 11.4 ml (155 mmol) Aceton liefert die Zielverbindung als farbloses Öl. Ausbeute: 13.0 g (57%); Massenspekrometrie: [M+H]⁺ = 193.

### b) N-[2-(4-Isopropyl-phenyl)-1,1-dimethyl-ethyl]-acetamid

Es wird eine Ritter-Reaktion mit 10.2 g (53 mmol) 1-(4-Isopropyl-phenyl-2-methylpropan-2-ol in derfür Beispiel 9b) beschriebenen Weise durchgeführt. Das Reaktionsgemisch wird auf Eiswasser gegossen und mit Natronlauge alkalisch gestellt, wobei ein Feststoff ausfällt. Dieser wird abgesaugt und getrocknet. Ausbeute: 9.90 g (80%); Massenspektrometrie: [M+H]⁺ = 234.

### c) 2-(4-Isopropyl-phenyl)-1,1-dimethyl-ethylamin

Umsetzung von 9.80 g (42 mmol) N-[2-(4-Isopropyl-phenyl)-1,1-dimethyl-ethyl]-acetamid in Analogie zur Vorschrift für Beispiel 9c). Ausbeute: 7.00 g (71%, Hydrochlorid); Smp. 202-206°C.

### d) 6-Benzyloxy-8-{1-hydroxy-2-[2-(4-isopropyl-phenyl)-1,1-dimethyl-ethylamino]-ethyl}-4H-benzo[1,4]oxazin-3-on

2.18 g (6.1 mmol) Benzyloxy-8-(2-ethoxy-2-hydroxy-acetyl)-4H-benzo[1,4]oxazin-3-on und 1:1 g (5.8 mmol) 2-(4-Isopropyl-phenyl)-1,1-dimethyl-ethylamin werden eine Stunde bei 50-80°C in 40 mL Ethanol gerührt. Nach Abkühlung auf Raumtemperatur werden 0.24 g (6.3 mmol) Natriumborhydrid zugesetzt. Man läßt eine Stunde rühren, verdünnt mit 5 mL Aceton und läßt weitere 30 Minuten rühren. Die Reaktionsmischung wird mit Salzsäure angesäuert, mit 100 mL Wasser und 80 mL Ethylacetat versetzt und mit Ammoniak alkalisch gestellt. Die organische Phase wird abgetrennt, mit Natriumsulfat getrocknet und vom Lösungsmittel befreit. Der Rückstand wird in 20 mL Ethylacetat und 10 mL Wasser gelöst, mit konz. Salzsäure sauer gestellt und mit Diethylether verdünnt. Nach Zugabe einer Kristallisationshilfe wird der ausfallende Feststoff abgesaugt und gewaschen. Weißer Feststoff. Ausbeute: 1.7 g (52 %, Hydrochlorid); Smp. 220-222°C.

### e) 6-Hydroxy-8-{1-hydroxy-2-[2-(4-isopropyl-phenyl)-1,1dimethyl-ethylamino]-ethyl}-4H-benzo[1,4]oxazin-3-on

1.6 g (3.0 mmol) 6-Benzyloxy-8-{1-hydroxy-2-[2-(4-isopropyl-phenyl)_{~}1,1-dimethylethylamino]-ethyl}-4H-benzo[1,4]oxazin-3-on werden in Methanol gelöst und mit Palladium auf Kohle als Katalysator bei Normaldruck und Raumtemperatur hydriert. Der Katalysator wird abgesaugt, das Lösungsmittel abdestilliert und der Rückstand in Isopropanol auskristallisiert. Weißer Feststoff. Ausbeute: 1.1 g (85%, Hydrochlorid); Smp. 248-250°C; Massenspektrometrie: [M+H]⁺ = 399.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 6: 8-{2-[2-(4-Ethyl-phenyl)-1,1-dimethyl-ethylamino]-1-hydroxy-ethyl}-6-hydroxy-4H-benzo[1,4]oxazin-3-on

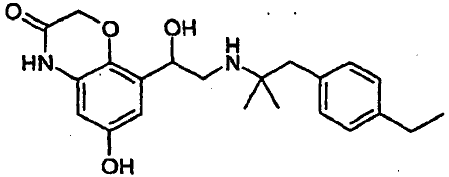

### a) 1-(4-Ethyl-phenyl)-2-methyl-propan-2-ol

Zu 39 mL einer 3 molaren Lösung von Methylmagnesiumbromid in Diethylether werden unter Kühlung mit dem Eisbad 14.8 g (90 mmol) 1-(4-Ethyl-phenyl)-propan-2-on, gelöst in Diethylether, so zugetropft, dass die Temperatur nicht über 30°C steigt. Nach beendeter Zugabe lässt man die Reaktionsmischung 1.5 Stunden refluxieren und hydrolisiert dann mit 10%iger Ammnoniumchlorid-Lösung. Nach Abtrennung der organischen Phase wird die wässrige Phase mit Diethylether extrahiert. Die vereinigten Etherphasen werden mit Wasser gewaschen, mit Natriumsulfat getrocknet und eingeengt. Das so erhaltene Öl wird direkt weiter umgesetzt. Ausbeute: 15.5 g (90%).

### b) N-[2-(4-Ethyl-phenyl)-1,1-dimethyl-ethyl]-acetamid

Zu 15.5 g (87 mmol) 1-(4-Ethyl-phenyl)-2-methyl-propan-2-ol in 4.8 mL (91 mmol) Acetonitril und 15 mL Eisessig werden innerhalb von 15 Minuten 6.2 mL konz. Schwefelsäure zugetropft, wobei die Temperatur auf 65°C ansteigt. Anschließend wird eine Stunde gerührt, mit Eiswasser verdünnt und mit konz. Natronlauge alkalisch gestellt. Nach weiteren Rühren über 30 Minuten wird derausgefallene Feststoff abgesaugt und mit Wassergewaschen. Das Rohprodukt wird in Ethylacetat gelöst, mit Natriumsulfat getrocknet und eingeengt. Das zurückbleibende Öl versetzt man mit Petrolether, wobei ein Feststoff ausfällt, der abfiltriert und getrocknet wird. Ausbeute: 16.3 g (85%); Schmp. 90-92°C.

### c) 2-(4-Ethyl-phenyl)-1,1-dimethyl-ethylamin

16.3 g (74 mmol) N-[2-(4-Ethyl-phenyl)-1,1-dimethyl-ethyl]-acetamid und 8.0 g Kaliumhydroxid werden über 15 Stunden in 60 mL Ethylenglykol unter Rückfluß erhitzt. Die Reaktionsmischung wird mit Eiswasser versetzt und dreimal mit Diethyletherextrahiert. Die vereinigten organischen Phasen werden mit Wasser gewaschen, mit Natriumsulfat getrocknet und vom Lösungsmittel befreit. Zur Herstellung des Hydrochlorids wird das Rohprodukt in Acetonitril gelöst und nacheinander mit etherischer Salzsäure und Diethylether versetzt. Der ausfallende Feststoff wird abgesaugt und getrocknet. Ausbeute: 11.0 g (69%, Hydrochlorid); Schmp. 165-167°C.

### d) 6-Benzyloxy 8-{2-[2-(4-ethyl-phenyl)-1,1-dimethyl-ethylamino]-1-hydroxy-ethyl}-4H-benzo[1,4]oxazin-3-on

Die Zielverbindung wird in Analogie zur Vorschrift für Beispiel 8d) aus 2.14 g (6.0 mmol) 6-Benzyloxy-8-(2-ethoxy-2-hydroxy-acetyl)-4H-benzo[1,4]oxazin-3-on und 1.0g(5.6 mmol)2-(4-Ethyl-phenyl)-1,1-dimethyl-ethylaminher-gestellt. Weißer Feststoff. Ausbeute: 1.7 g (54%, Hydrochlorid); Smp. 210-214°C.

### e) 8-{2-[2-(4-Ethyl-phenyl)-1,1 1-dimethyl-ethylamino]-1-hydroxy-ethyl}-6-hydroxy-4H-benzo[1,4]oxazin-3-on

Die Hydrogenolyse von 1.45 g (2.75 mmol) 6-Benzyloxy-8-(2-[2-(4-ethyl-phenyl)-1,1-dimethyl-ethylamino]-1-hydroxy-ethyl}-4H-benzo[1,4]oxazin-3-on nach der Vorschrift für Beispiel 8e) liefert die Zielverbindung in Form eines weißen Feststoffs. Ausbeute: 1.07 g (92%; Hydrochlorid); Smp. 266-269°C; Massenspektrometrie: [M+H]⁺ = 385.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 7: 8-{2-[2-(4-Fuoro-3-methyl-phenyl)-1,1-dimethyl-ethylamino]-1-hydroxyethyl}-6-hydroxy-4H-benzo[1,4] oxazin-3-on

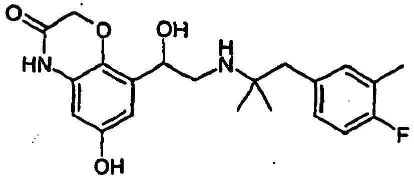

### a) 1-Fluor-2-methyl-4-(2-methyl-propenyl)-benzol

100 mL einer 0.5 molaren Lösung von 4-Fluor-3-methyl-phenylmagnesiumbromid in THF werden innerhalb von 30 Minuten mit 4.7 mL (50 mmol) Isopropylaldehyd versetzt, wobei die Temperatur auf 45°C ansteigt. Es wird 30 Minuten gerührt, 1 Stunde refluxiert und dann mit 10%iger Ammoniumchlorid-Lösung hydrolisiert. Nach Abtrennung der organischen Phase extrahiert man mit Diethylether. Die organischen Phasen werden vereinigt, getrocknet und eingeengt. Der so erhaltene Alkohol wird in 100 mL Toluol gelöst, mit 1 g
p-Toluensulfonsäure Monohydrat versetzt und drei Stunden am Wasserabscheider unter Rückfluß erwärmt. Die Reaktionsmischung wird auf Wasser gegossen und mit konz. Natronlauge alkalisch gestellt. Nach Abtrennung der organischen Phase wird diese mit Wasser gewaschen, mit Natriumsulfat getrocknet und vom Lösungsmittel befreit. Eine fraktionierte Destillation des Rückstandes liefert das Produkt in Form einer farblosen Flüssigkeit (Sdp. 80-85°C/10 mbar). Ausbeute: 4.1 g (50%).

### b) N-[2-(4-Fluor-3-methyl-phenyl)-1,1-dimethyl-ethyl]-formamid

Zu 1.5 g (31 mmol) Natriumcyanid in 5 mL Eisessig werden bei 5-15°C 4.9 mL konz. Schwefelsäure getropft. Anschließend wird die Mischung mit 3.9 g (24 mmol) 1-Fluor-2-methyl-4-(2-methyl-propenyl)-benzen, gelöst in 10 mL Eisessig, versetzt und 1 Stunde bei 50-60°C gerührt. Die Reaktionsmischung wird mit Eiswasser verdünnt, mit konz. Natronlauge alkalisch gestellt und mit Dichlormethan extrahiert. Die organische Phase wird mit Natriumsulfat getrocknet und im Vakuum vom Lösungsmitteln befreit. Das so erhaltene leicht gelbe Öl wird direkt weiter umgesetzt. Ausbeute: 4.3 g (87%).

### c) 2-(4-Fluor-3-methyl-phenyl)-1,1-dimethyl-ethylamin

4.3 g (20.6 mmol) N-[2-(4-Fluor-3-methyl-phenyl)-1,1-dimethyl-ethyl]-formamid, 20 mL konz. Salzsäure und 20 mL Wasser werden über 2 Stunden unter Rückfluß erwärmt. Man verdünnt die Reaktionsmischung mit Wasser, stellt mit konz. Natronlauge alkalisch und extrahiert mit Dichlormethan. Die organischen Phasen werden mit Natriumsulfat getrocknet und eingeengt. Der Rückstand wird in Ethylacetat gelöst, mit etherischer Salzsäure versetzt und gekühlt. Die ausfallenden Kristalle werden abgesaugt und mit Diethylether gewaschen und getrocknet. Weißer Feststoff. Ausbeute: 3.9 g (87%, Hydrochlorid); Schmp. 196-198°C.

### d) 6-Benzyloxy-8-{2-[2-(4-fluor-3-methyl-phenyl)-1,1-dimethyl-ethylamino]-1-hydroxyethyl}-4H-benzo[1,4]oxazin-3-on

1.10 g (3.1 mmol) Benzyloxy-8-(2-ethoxy-2-hydroxy-acetyl)-4H-benzo[1,4]oxazin-3-on und 0.50 g (2.8 mmol) 2-(4-Fluor-3-methyl-phenyl)-1,1-dimethyl-ethylamin werden anlog zu der Vorschrift für Beispiel 8d) umgesetzt und aufgearbeitet. Weißer Feststoff. Ausbeute: 0.75 g (47%, Hydrochlorid); Smp. 228-230°C.

### e) 8-{2-[2-(4-Fluoro-3-methyl-phenyl)-1,1-dimethyl-ethylaminol-1-hydroxy-ethyl}-6-hydroxy-4H-benzo[1,4]oxazin-3-on

Die Hydrierung von 0.70 g (1.4 mmol) 6-Benzyloxy-8-{2-[2-(4-fluor-3-methyl-phenyl}-1,1-dimethyl-ethylamino]-1-hydroxy-ethyl}-4H-benzo[1,4]oxazin-3-on liefert die Zielverbindung als weißen Feststoff. Ausbeute: 0.50 g (87%, Hydrochlorid); Smp. 278-280°C; Massenspektroskopie: [M+H]⁺ = 389.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 8: 8-{2-[2-(4-Fluor-2-methyl-phenyl)-1,1-dimethyl-ethylaminol-1-hydroxyethly}-6-hydroxy-4H-benzo[1,4] oxazin-3-on

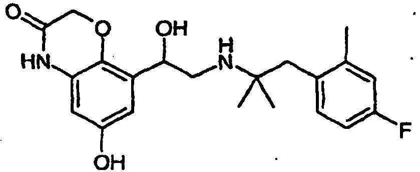

### a) Essigsäure-1-(4-fluor-2-methyl-phenyl)-2-methyl-propyl ester

500 mL einer 0.5 molaren Lösung von 4-Fluor-6-methylphenylmagnesiumbromid und 23.2 mL (260 mmol) Isopropylaldehyd werden analog zu Beispiel 10a) umgesetzt. Nach Hydrolyse mit 10%iger Ammoniumchlorid-Lösung wird die wässrige Phase abgetrennt und mit Diethylether extrahiert. Die vereinigten organischen Phasen werden mit Natriumsulfat getrocknet und eingeengt. Der so erhaltene Alkohol wird dann in 50 mL Acetanhydrid gelöst, mit 1 mL konz. Schwefelsäure versetzt und drei Stunden unter Rückfluß gerührt. Dann wird die Reaktionsmischung auf Wasser gegossen, eine weitere Stunde gerührt und alkalisch gestellt. Man extrahiert mit Dichlormethan, trocknet die organischen Phasen mit Natriumsulfat und destilliert die Lösungsmittel ab. Eine fraktionierte Destillation des Rückstands liefert das Produkt in Form einer farblosen Flüssigkeit (Sdp. 105-110°C/8 mbar). Ausbeute 29.0 g (52%).

### b) N-[2-(4-Fluor-2-methyl-phenyl)-1,1-dimethyl-ethylformamid

29.0g(130 mmol)Essigsäure-1-(4-fluor-2-methyl-phenyl)-2-methyl-propyl ester werden analog zur Vorschrift für Beispiel 10b) umgesetzt und aufgearbeitet. Gelbes Öl. Ausbeute: 27.0 g (99%).

### c) 2-(4-Fluor-2-methyl-phenyl)-1,1-dimethyl-ethylamin

Zur Herstellung des Amins werden 27.0 g (130 mmol) N-[2-(4-Fluor-2-methyl-phenyl)-1,1-dimethyl-ethyl]-formamid wie in der Vorschrift für Beispiel 10c) beschrieben umgesetzt. Weißer Feststoff. Ausbeute: 15.5 g (55%, Hydrochlorid); Smp. 277-280°C.

### d 6-Benzyloxy-8-{2-[2-[4-fluor-2-methyl-phenyl)-1,1-dimethyl-ethylamino]-1-hydroxyethyl)-4H-benzo[1,4]oxazin-3-on

Darstellung in Analogie zur Vorschrift für Beispiel 8d) aus 0.95 g (2.66 mmol) Benzyloxy-8-(2-ethoxy-2-hydroxy-acetyl)-4H-benzo[1,4]oxazin-3-on und 0.43 g (2.37 mmol) 2-(4-Fluor-2-methyl-phenyl)-1,1-dimethyl-ethylamin. Ausbeute: 0.75 g (55%, Hydrochlorid); Smp. 233-236°C.

### e) 8-{2-[2-(4-Fluor-2-methyl-phenyl)-1,1-dimethyl-ethylamino1-1-hydroxy-ethyl}-6-hydroxy-4H-benzo[1,4]oxazin-3-on

Die Debenzylierung von 0.70 g (1.36 mmol) 6-Benzyloxy-8-{2-[2-[4-fluor-2-methylphenyl)-1,1-dimethyl-ethyl-amino]-1-hydroxy-ethyl}-4H-benzo[1,4]oxazin-3-on liefert die Zielverbindung in Form eines weißen Feststoffs. Ausbeute: 0.50 g (87%, Hydrochlorid); Smp. 278-280°C; Massenspektroskopie: [M+H]⁺ = 389.

Die (R)- und (S)-Enantiomere dieses Ausführungsbeispiels können durch Auftrennung des Racemats in Analogie zu gängigen, im Stand der Technik bekannten Racematspaltungs methoden erhalten werden.

### Beispiel 4: 8-{2-[2-(2,4-Difluor-phenyl)-1,1-dimethyl-ethylamino]-1-hydroxy-ethyl}-6-hydroxy-4H-benzo[1,4]oxazin-3-on

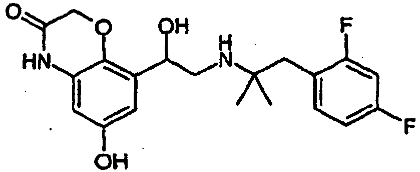

### a) 1-(2,4-Difluor-phenyl)-2-methyl-propan-2-ol

Zu einer Lösung von 500 mL 0.25 molarem 2,4-Difluorbenzylmagnesiumbromid in Diethylether werden inner-halb von 20 Minuten 11.0 mL Aceton, verdünnt mit 50 mL Diethylether, zugetropft. Anschließend wird 1.5 Stunden unter Rückfluß gerührt und dann mit 10%iger Ammoniumchlorid-Lösung hydrolysiert. Die Etherphase wird abgetrennt, mit Wasser gewaschen, mit Natriumsulfat getrocknet und eingeengt. Die fraktionierte Destillation des Rückstands liefert den Alkohol als farblose Flüssigkeit (Sdp. 70-73°C/ 2 mmbar). Ausbeute: 20.0 g (86%).

### b) N-[2-(2 4-Difluor-phenyl]-1,1-dimethyl-ethyl]-formamid

Ritter-Reaktion mit 20 g (110 mmol) 1-(2,4-Difluor-phenyl)-2-methyl-propan-2-ol nach dem für Beispiel 10b) beschriebenen Verfahren. Gelbes Öl. Ausbeute: 22.0 g (94%).

### c) 2-(2,4-Difluor-phenyl)-1,1-dimethyl-ethylamin

Umsetzung von 22.0 g (100 mmol) N-[2-(2,4-Dmuor-phenyl]-1,1-dimethyl-ethyl]-formamid in Analogie zur Vorschrift für Beispiel 10c). Ausbeute: 16.0 g (72%, Hydrochlorid); Smp.201-203°C.

### d) 6-Benzyloxy 8-{2-[2-(2,4-difluor-phenyl)-1,1-dimethyl-ethylaminol]-1-hydroxy-ethyl}-4H-benzo[1,4]oxazin-3-on

Umsetzung von 0.89 g (2.49 mmol) Benzyloxy-8-(2-ethoxy-2-hydroxy-acetyl)-4H-benzo[1,4]oxazin-3-on und 0.40 g (2.16 mmol) 2-(2,4-Difluor-phenyl)-1,1-dimethylethylamin in der für Beispiel 8d) beschriebenen Weise. Ausbeute: 0.80 g (62%, Hydrochlorid); Smp. 245-247°C.

### e) 8-{2-[2-(2,4-Difluor-phenyl)-1,1-dimethyl-ethylamino]-1-hydroxy-ethyl}-6-hydrory-4H-benzo[1,4]oxazin-3-on

Die Hydrogenolyse von 0.70 g (1.35 mmol) 6-Benzyloxy-8-(2-[2-(2,4-difluor-phenyl)-1,1-dimethyl-ethylamino]-1-hydroxy-ethyl)-4H-benzo[1,4]oxazin-3-on liefert die Zielverbindung als weißen Feststoff. Ausbeute: 0.48 g (83%, Hy-drochlorid); Smp. 279-280°C; Massenspektroskopie: [M+H]⁺ = 393.

Die (R)- und (S)-Enantiomere dieses Ausführungsbeispiels können durch Auftrennung des Racemats in Ana-logie zu gängigen, im Stand der Technik bekannten Racematspältungsmethoden erhalten werden.

### Beispiel 5: 8-{2-[2-(3,5-Difluor-phenyl)-1,1-dimethyl-ethylaminol-1-hydroxy-ethyl}-6-hydroxy-4H-benzo[1,4]oxazin-3-on

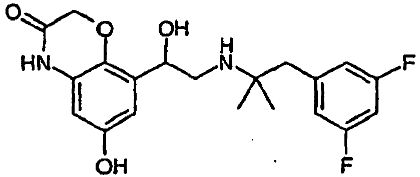

### a) 1-(3,5-Difluor-phenyl)-2-methyl-propan-2-ol

Die Zielverbindung wird durch Umsetzung einer Grignardverbindung, hergestellt aus 25.0 g (121 mmol) 3,5-Difluorbenzylbromid, mit 12.6 mL (171 mmol) Aceton erhalten. Gelbes Öl. Ausbeute: 13.5 g (60%).

### b) 2-(3.5-Difluor-phenyl)-1,1-dimethyl-ethylamin

Die Ritter-Reaktion von 5.5 g (29.5 mmol) 1-(3,5-Difluor-phenyl)-2-methyl-propan-2-ol und 1.8g Natriumcyanid liefert 7.0 g Formamid, das zur Abspaltung der Formylgruppe mit Salzsäure behandelt wird. Leicht gelbes Öl. Ausbeute: 4.6 g (75%).

### c) 6-Benzyloxy-8-{2-[2-(3,5-difluor-phenyl)-1,1-dimethyl-ethylamino]-1-hydroxy-ethyl}-4H-benzo[1,4] oxazin-3-on

Darstellung aus 1.73 g (4.84 mmol) Benzyloxy-8-(2-ethoxy-2-hydroxy-acetyl)-4H-benzo[1,4]oxazin-3-on und 0.80 g (4.32 mmol) 2-(3,5-Difluor-phenyl)-1,1-dimethylethylamin in der üblichen Weise. Ausbeute: 1.50 g (58 %, Hydro-chlorid); Smp. 240-244°C.

### d) 8-{2-[2-(3,5-Difluor-phenyl)-1,1-dimethyl-ethylamino]-1-hydroxy-ethyl}-6-hydroxy-4H-benzo[1,4]oxazin-3-on

Hydrogenolyse von 1.30 g (2.43 mmol) 6-Benzyloxy-8-(2-[2-(3,5-difluor-phenyl)-1,1-dimethyl-ethylamino]-1-hydroxy-ethyl}-4H-benzo[1,4]oxazin-3-on liefert die Zielverbindung als weißen Feststoff. Ausbeute: 0.90 g (86%, Hydrochlorid); Smp. 150-158°C; Massenspektroskopie: [M+H]⁺ = 393.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Beispiel 6: 8-{2-[2-(4-Ethoxy-phenyl)-1,1-dimethyl-ethylamino]-1-hydroxy-ethyl}-6-hydroxy-4H-benzo[1,4]oxazin-3-on

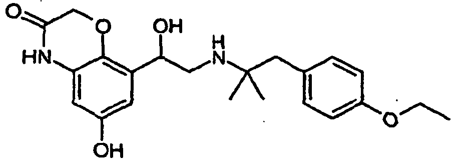

### a) [2-(4-Ethoxy-phenyl)-1,1-dimethyl-ethyl]-carbaminsäure benzyl ester

15.0 g (50 mmol) [2-(4-Hydroxy-phenyl)-1,1-dimethyl-ethyl]-carbaminsäure benzyl ester werden mit 7.5 mL (92 mmol) Ethyliodid und 21 g (150 mmol) Kaliumcarbonat über 10 Stunden bei 90-100°C gerührt. Die Reaktionsmischung wird mit Ethylacetat versetzt, zweimal mit Wasser gewaschen und mit Natriumsulfat getrocknet. Nach dem Abdestillieren der Lösungsmittel bleibt ein gelbes Öl (15.0 g, 92%) zurück, das direkt weiter umgesetzt wird.

### b) 2-(4-Ethoxy-phenyl)-1,1-dimethyl-ethylamin

Eine Lösung von 15.0 g (49 mmol) [2-(4-Ethoxy-phenyl)-1,1-dimethyl-ethyl]-carbaminsäure benzyl ester in 100 mL Eisessig wird mit 2 g Palladium auf Kohle (10%ig) versetzt und anschließend bei 5 bar und 40 bis 50°C hydriert. Der Katalysator wird abfiltriert und das Filtrat vom Lösungsmittel befreit. Der Rückstand wird in wenig Wasser gelöst, mit konz. Natronlauge alkalisch gestellt und mit Ethylacetat extrahiert. Die organische Phase wird mit Wasser gewaschen, mit Natriumsulfat getrocknet und eingeengt. Das Rohprodukt wird in Acetonitril gelöst und mit etherischer Salzsäure angesäuert. Der nach Zugabe von Diethylether ausfallende Feststoff wird abgesaugt und getrocknet. Ausbeute: 8.8 g (Hydrochlorid, 84%); Schmp. 198-200°C.

### c) 6-Benzyloxy-8-{2-[2-(4-ethoxy_phenyl)-1,1-dimethyl-ethylaminol-1-hydroxy-ethyl}-4H-benzo[1,4]oxazin-3-on

2.14 g (6.0 mmol) 6-Benzyloxy-8-(2-ethoxy-2-hydroxy-acetyl)-4H-benzo[1,4]oxazin-3-on und 1.0 g (5.2 mmol) 2-(4-Ethoxy-phenyl)-1,1-dimethyl-ethylamin werden in 40 mL Ethanol eine Stunde bei 50-80°C gerührt. Nach Abkühlung auf Raumtemperatur werden 0.23 g (6.0 mmol) Natriumborhydrid zugegeben und man läßt über eine weitere Stunde rühren. Die Reaktionsmischung wird mit 5 ml Aceton versetzt, 30 Minuten gerührt, mit Eisessig angesäuert und eingeengt. Der Rückstand wird mit Wasser und Ethylacetat versetzt und alkalisch gestellt. Die organische Phase wird abgetrennt, mit Wasser gewaschen, mit Natriumsulfat getrocknet und im Vakuum vom Lösungsmittel befreit. Der Rückstand wird wieder in Ethylacetat und Wasser gelöst, mit konz. Salzsäure versetzt und mit Diethylether verdünnt. Der ausfallende Feststoff wird abgesaugt und mit Diethylethergewaschen. Weißer Feststoff. Ausbeute: 2.0 g (61 %, Hydrochlorid); Smp. 214-216°C.

### d) 8-{2-[2-(4-Ethoxy-phenyl)-1,1-dimethyl-ethylaminol-1-hydroxy-ethyl}-6-hydroxy-4H-benzo[1,4]oxazin-3-on

1.5 g (2.8 mmol) 6-Benzyloxy-8-{2-[2-(4-ethoxy-phenyl)-1,1-dimethyl-ethylamino]-1-hydroxy-ethyl}-4H-benzo [1,4]oxazin-3-on in 80 mL Methanol werden mit 250 mg Palladium auf Kohle (10 %ig) als Katalysator bei Raumtemperatur und Normaldruck hydriert. Der Katalysator wird abgesaugt und das Filtrat eingeengt. Der Rückstand wird in 5 mL Ethanol durch Erwärmen gelöst, angeimpft und mit Ethylacetat verdünnt. Der ausfallende Feststoff wird abfiltriert und gewaschen. Weißer Feststoff. Ausbeute 1.0 g (83%, Hydrochlorid); Smp. 232-235°C; Massenspektrometrie: [M+H]⁺ = 401.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 9: 8-{2-[2-(3,5-Dimethyl-phenyl)-1,1-dimethyl-ethylamino]1-hydroxy-ethyl}-6-hydroxy-4H-benzo[1,4]oxazin-3-on

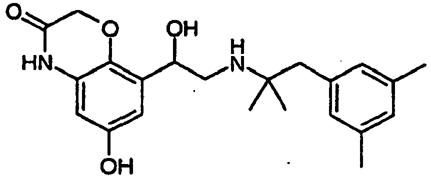

### a) 1-(3,5-Dimethyl-phenyl)-2-methyl-propanol-2-ol

Erhalten aus der Umsetzung von (3,5-Dimethyl-phenyl)-essigsäureethylester mit Methylmagnesiumbromid.

### b) 2-(3,5-Dimethyl-phenyl)-1,1-dimethyl-ethylamin

Durch Umsetzung von 6.00 g (34 mmol) 1-(3,5-Dimethyl-phenyl)-2-methyl-propanol-2-ol und 2.00 g (41 mmol) Natriumcyanid in einer Ritter-Reaktion werden 2.40 g 2-(3,5-Dimethyl-phenyl)-1,1-dimethyl-ethylformamid (35% Ausbeute) erhalten. Zur Freisetzung des Amins wird das Formamid (2.40 g, 11.7 mmol) mit Salzsäure behandelt. Die Durchführung und Aufarbeitung erfolgen in Analogie zur Vorschrift für Beispiel 10c). Öl. Ausbeute: 1.70 g (82%); Massenspektroskopie: [M+H]⁺ = 178.

### c) 6-Benzyloxy-8-{2-[2-(3,5-dimethyl-phenyl)-1,1-dimethyl-ethylamino]-1-hydroxyethyl}-4H-benzo[1,4]oxazin-3-on

Darstellung in Analogie zur Vorschrift für Beispiel 8d) aus 1.47 g (4.1 mmol) Benzyloxy-8-(2-ethoxy-2-hydroxy-acetyl)-4H-benzo[1,4]oxazin-3-on und 0.65 g (3.7 mmol) 2-(3,5-Dimethyl-phenyl)-1,1-dimethyl-ethylamin. Ausbeute: 1.1 g (51 %, Hydrochlorid); Smp. 220-222°C.

### d) 8-{2-[2-(3,5-Dimethyl-Phenyl)-1,1-dimethyl-ethylamino]-1-hydroxy-ethyl}1-6-hydroxy-4H-benzo[1,4]oxazin-3-on

Die Zielverbindung wurde nach Hydrogenolyse von 0.90 g (1.71 mmol) 6-Benzyloxy-8-{2-[2-(3,5-dimethylphenyl)-1,1-dimethyl-ethylamino]-1-hydroxy-ethyl}-4H-benzo[1,4]oxazin-3-on und Umkristallisation des Rohproduktes aus Isopropanol erhalten. Weißer Feststoff. Ausbeute: 0.50 g (69%, Hydrochlorid); Smp. 235-238°C; Massenspektroskopie: [M+H]⁺ = 385.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 10: 4-(4-{2-[2-Hydroxy-2-(6-hydroxy-3-oxo-3,4-dihydro-2H-benzo[1,4]oxazin-8-yl)-ethylamino]1-2-methylpropyl}-phenoxy)-buttersäure

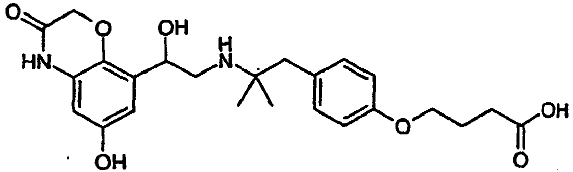

### a) 4-[4-(2-Amino-2-methyl-propyl)-phenoxy]-buttersäureethylester

4.5 g (15.0 mmol) [2-(4-Hydroxy-phenyl)-1,1-dimethyl-ethyl]-carbaminsäure benzyl ester, 2.3 mL (16.0 mmol) 4-Brom-buttersäureethylester, 2.3 g (16.6 mmol) Kaliumcarbonat und 0.3 g (1.8 mmol) Kaliumiodid in 20 mL Dimethylformamid werden 13 h Stunden bei 120°C erwärmt. Die Reaktionsmischung wird mit Ethylacetat verdünnt und nacheinander mit Wasser, Natriumhydroxid-Lösung und Wasser gewaschen. Die organische Phase wird mit Natriumsulfat getrocknet und eingeengt. Den Rückstand reinigt man chromatographisch (Laufmittel: CyclohexanlEthylacetat = 9:1). Es werden 5.0 g eines gelben Öls isoliert, das in 50 mL Essigsäure gelöst wird und mit 1.0 g Palladium auf Kohle als Katalysator bei 40°C und 3 bar hydriert wird. Der Kataysator wird abfiltriert und das Filtrat vom Lösungsmitttel befreit. Der Rückstand wird in Diethylether gelöst und mit etherischer Salzsäure versetzt. Der ausfallende Feststoff wird abgesaugt und getrocknet. Ausbeute: 2.9 g (66% über zwei Stufen, Hydrochlorid); Smp. = 103-105°C.

### b) 4-(4-{2-[2-(6-Benzyloxy-3-oxo-3,4-dihydro-2H-benzo(1,4]oxazin-8-yl)-2-hydroxy-ethylamino]-2-methyl-propyl}-phenoxy)-buttersäureethylester

1.20 g (3.36 mmol) Benzyloxy-8-(2-ethoxy-2-hydroxy-acetyl)-4H-benzo[1,4]oxazin-3-on und 0.90 g (3.22 mmol) 4-[4-(2-Amino-2-methyl-propyl)-phenoxy]-buttersäureethylester werden in der für Beispiel 8d) beschriebenen Weise umgesetzt. Das Rohprodukt wird in 10 mL Ethylacetat und 10 mL Wasser gelöst und unter Rühren mit Oxalsäure versetzt. Die Lösung verdünnt man mit Diethylether und der ausfallende Feststoff wird abgesaugt und mit Diethylether gewaschen. Ausbeute: 1.20 g (54%, Oxalat); Smp. 223-227°C.

### c) 4-(4-{2-[2-(6-Benzyloxy-3-oxo-3,4-dihydro-2H-benzo[1,4]oxazin-8-yl)-2-hydroxyethylamino]-2-methyl-propyl}-phenoxy)-buttersäure

Eine Lösung von 1.00 g (1.73 mmol) 4-(4-{2-[2-(6-Benzyloxy-3-oxo-3,4-dihydro-2H-benzo[1,4]oxazin-8-yl)-2-hydroxy-ethylamino]-2-methyl-propyl}-phenoxy)-buttersäureethylester in 25 mL Methanol wird mit 2.5 mL 1 N Natrium-hydroxid-Lösung versetzt, 30 Minuten refluxiert und dann mit 1 N Salzsäure neutralisiert. Die Lösung wird eingeengt und das zurückbleibende Öl durch Erwärmen in 5 mL n-Butanol gelöst. Nach Zugabe einer Kristallisationshilfe fällt ein Feststoff aus, der abgesaugt und mit Aceton und Diethylether gewaschen wird. Ausbeute: 0.75 g (79%); Smp. 216-218°C.

### d) 4-(4-{2-[2-Hydroxy-2-(6-hydroy-3-oxo-3,4-dihydro-2H-benzo[1,4]oxazin-8-yl)-ethylamino]-2-methyl-propyl}-phen-oxy)-buttersäure

0.70 g (1.28 mmol) 4-(4-{2-[2-(6-Benzyloxy-3-oxo-3,4-dihydro-2H-benzo[1,4]oxazin-8-yl)-2-hydroxy-ethylami-no]-2-methyl-propyl}-phenoxy)-buttersäure werden in 25 mL Methanol und 2 mL Essigsäure gelöst und in Gegenwart von 150 mg Palladium auf Kohle (10%ig) bei Raumtemperatur und Normaldruck hydriert. Der Katalysator wird abfiltriert und das Filtrat vom Lösungsmittel befreit. Das Produkt erhält man durch Kristallisation aus einem Methanol/Aceton-Gemisch. Ausbeute: 0.40 g (68%); Smp. 201-204°C; Massenspektroskopie: [M+H]⁺ = 459.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 11: 8-{2-[2-(3,4-Difluor-phenyl)-1,1-dimethyl-ethylamino]-1-hydroxy-ethyl}-6-hydroxy-4H-benzo[1,4]oxazin-3-on

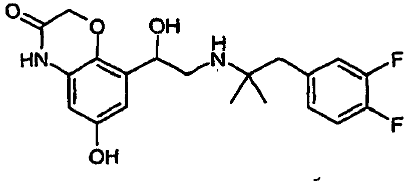

### a) 1-(3,4-Difluor-phenyl)-2-methyl-propan-2-ol

Aus 23.0 g (111 mmol) 3,4-Difluorbenzylbromid wird ein Grignard hergestellt, den man dann mit 11.6 mL (158 mmol) Aceton umsetzt. Leicht gelbes Öl. Ausbeute: 9.7 g (47%); R_{f}-Wert: 0.55 (Ethylacetat/Petrolether = 1:3).

### b) N-[2-(3,4-Difluor-phenyl)-1,1-dimethyl-ethyl]-formamid

Die Zelverbindung wird über eine Ritter-Reaktion mit 4.0 g (21.5 mmol) 1-(3,4-Difluorphenyl)-2-methyl-propan-2-ol erhalten. Leicht gelbes Öl. Ausbeute: 4.0 g (87%); Massenspektrometrie: [M+H]⁺ = 214.

### c) 2-(3,4-Difluor-phenyl)-1,1-dimethyl-ethylamin

4.00 g (18.5 mmol) N-[2-(3,4-Difluor-phenyl)-1,1-dimethyl-ethyl]-formamid werden in Ethanol gelöst, mit konz. Salzsäure versetzt und über Nacht unter Rückfluß erwärmt. Die Reaktionslösung wird auf Eiswasser gegossen, mit Natriumhydroxid alkalisch gestellt und mit tert-Butylmethylether extrahiert. Die organischen Phasen werden mit Wasser gewaschen, mit Natriumsulfat getrocknet und eingeengt. Gelbes Öl. Ausbeute: 3.2 g (92%); Massenspektrometrie: [M+H]⁺ = 186.

### d) 8-{2-[2-(3,4-Difiuor-phenyl)-1,1-dimethyl-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 185 mg (1 mmol) 2-(3,4-Difluor-phenyl)-1,1-dimethyl-ethylamin werden 30 Minuten in 5 mL Tetrahydrofuran bei Raumtemperaturgerührt. Man kühlt auf 0°C ab und tropft unter Argonatmosphäre 1.5 mL einer 2 molaren Lösung von Lithiumborhydrid in Tetrahydrofuran zu. Es wird 30 min bei Raumtemperatur gerührt, mit 10 mL Dichlormethan und 3 mL Wasser versetzt, eine weitere Stunde gerührt und dann über Extrelut^{®} filtriert. Das das Ethanolamin enthaltende Eluat wird vom Lösungsmittel befreit. Der Rückstand wird in Methanol gelöst und mit Palladium auf Kohle (10%ig) als Katalysator bei 2.5 bar und Raumtemperatur hydriert. Anschließend wird der Katalysator abgetrennt und das Rohprodukt chromatographisch gereinigt. Weißer Feststoff. Ausbeute: 31 mg (6%, Trifluorethylacetat); Massenspektroskopie: [M+H]+ = 393.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 12: 8-{2-[2-(2-Chlor-4-fluor-phenyl)-1,1-dimethyl-ethylamino]-1-hydroxyethyl}-6-hydroxy-4H-benzo[1,4] oxazin-3-on

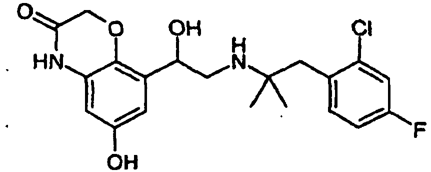

### a) 1-(2-Chlor-4-fluor-phenyl)-2-methyl-propan-2-ol

Herstellung aus 20 g (97 mmol) (2-Chlor-4-fluor-phenyl)-essigsäuremethylester und 98 mL einer 3 molaren Lösung von Methylmagnesiumbromid in Analogie zur Vorschrift für Beispiel 8a).

### b) N-[2-(2-Chlor-4-fluor-phenyl)-1,1-dimethyl-ethyl]-formamid

7.5 g (37 mmol) 1-(2-Chlor-4-fluor-phenyl)-2-methyl-propan-2-01 wurden nach der für Beispiel 10b) beschriebenen Vorschrift umgesetzt und aufgearbeitet. Das so erhaltene Öl wurde zur weiteren Reinigung an einer kurzen Kieselgelsäule chromatographiert (Petrolether/Ethylacetat = 9:1). 01. Ausbeute 7.4 g (87%); Massenspektrometrie: [M+H]⁺ = 230/232.

### c) 2-(2-Chlor-4-fluor-phenyl)-1,1-dimethyl-ethylamin

Umsetzung von 7.4 g (32 mmol) 14-[2-(2-Chlor-4-fluor-phenyl)-1,1-dimethyl-ethyl]-formamid wie in der Vorschrift für Beispiel 17c) beschrieben. Braunes Öl. Ausbeute: 5.14 g (79%); Massenspektrometrie: [M+H]⁺ = 202/204.

### d) 8-{2-[2-(2-Chlor-4-fluor-phenyl)-1,1-dimethyl-ethylamino]-1-hydroxy-ethyl}-6-hydroxy-4H-benzo[1,4]oxazin-3-on

357 mg (1 mmol) 6-Benzyloxy-8-(2-ethoxy-2-hydroxy-acetyl)-4H-benzo[1,4]oxazin-3-on und 202 mg (1 mmol) 2-(2-Chlor-4-fluor-phenyl)-1,1-dimethyl-ethylamin werden in Analogie zurVorschrift für Beispiel 10d) mit Lithiumborhydrid umgesetzt. Zur Debenzylierung des so erhaltenen Ethanolamins wird dieses in 3 mL Dichlormethan gelöst und auf-78°C abgekühlt. Bei dieser Temperatur tropft man 2 ml einer 1 molaren Lösung von Bortribromid in Dichlormethan zu und läßt langsam auf Raumtemperatur erwärmen. Die Reaktionsmischung wird mit 10 mL Dichlormethan und 3 mL Wasser versetzt und über Extrelut^{®} filtriert. Das Eluat wird vom Lösungsmittel befreit und der Rückstand mittels Chromatographie gereinigt. Weißer Feststoff. Ausbeute: 70 mg (13%, Trifluorethylacetat); Massenspektroskopie: [M+H]⁺ = 409/11.

Die (R)- und (S)-Enantiomere dieses Ausführungsbeispiels können durch Auftrennungdes Racemats in Analogie zu gängigen, im Stand der Technik bekannten Racematspaltungsmethoden erhalten werden.

### Vergleichsbeispiel 13: 8-{2-[2-(4-Chlor-phenyl)-1,1-dimethyl-ethylamino)-1-hydroxy-ethyl}-6-hydroxy-4H-benzo[1,4]oxazin-3-on

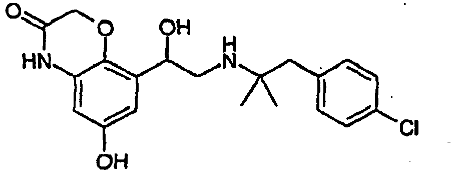

Eine Lösung von 300 mg (0.91 mmol) 6-Benzyloxy-8-(2,2-dihydroxy-acetyl)-4H-benzo[1,4]oxazin-3-on und 200 mg (1.09 mmol) 2-(4-Chlor-phenyl)-1,1-dimethylethylamin in 3 mL Ethanol wurde mit Molsieb versetzt und 90 Minuten bei 80°C gerührt. Man ließ auf Raumtemperatur abkühlen, fügte 35 mg (0.91 mmol) Natriumborhydrid hinzu und ließ 1 Stunde rühren. Anschließend wurde die Reaktionsmischung mit Natriumhydrogencarbonat-Lösung versetzt und mit Ethylacetat extrahiert. Die vereinigten organischen Phasen wurden vom Lösungsmittel befreit und der Rückstand chromatographiert (Laufmittel: Hexan/Ethylacetat/Methanol), wobei 305 mg Ethanolamin gewonnen werden konnten. Dieses wurde in 3 mL Dichlormethan gelöst und unter Argonatmosphäre auf -78°C abgekühlt. Es wurden 3 mL einer 1 molaren Lösung von Bortribromid in Dichlormethan zugetropft und man ließ eine Stunde bei -78°C und 20 Minuten bei Raumtemperatur Rühren. Dann tropfte man bei -78°C 3 mL konz. Ammoniaklösung zu und ließ 5 Minuten rühren. Die Reaktionsmischung wurde mit Ammoniumchlorid-Lösung versetzt und mit Ethylacetat extrahiert. Die vereinigten organischen Phasen wurden eingeengt und der Rückstand zur weiteren Reinigung chromatographiert (Kieselgel; Laufmittel: Dichlormethan/Methanol +1% Ammoniak). Beigefarbener Feststoff: 93 mg (26%); Massenspektrometrie: [M+H]⁺ = 391.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Vergleichsbeispiel 14: 8-{2-[2-(4-Brom-phenyl)-1,1-dimethyl-ethylarmino]-1-hydroxy-ethyl}-6-hydroxy-4H-benzo[1,4]oxazin-3-on

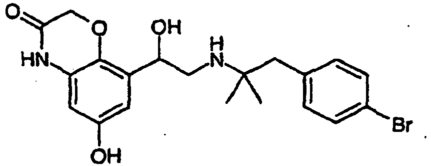

Die Ethanolamindarstellung und Debenrylierung erfolgten in der für Beispiel 19 beschriebenen Weise aus 300 mg (0.91 mmol) 6-Benzyloxy-8-(2,2-dihydroxy-acetyl)-4H-benzo[1,4]oxazin-3-on und 250 mg (1.09) mmol) 2-(4-Brom-phenyl)-1,1-dimethylethylamin. Beigefarbener Feststoff. Ausbeute: 54 mg (14%); Massenspektrometrie: [M+H]⁺ = 435, 437.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

### Beispiel 7: 8-{2-[2-(4-Fluor-phenyl)-1,1-dimethyl-ethylamino]-1-hydroxy-ethyl}-6-hydroxy-4H-benzo[1,4]oxazin-3-on

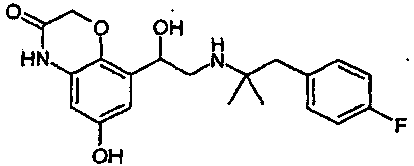

300 mg (0.91 mmol) 6-Benzyloxy-8-(2,2-dihydroxy-acetyl)-4H-benzo[1,4]oxazin-3-on und 183 mg (1.09 mmol) 2-(4-Fluor-phenyl)-1,1-dimethyl-ethylamin wurden in 3 ml Ethanol gelöst. Es wurde Molsieb zugegeben und 30 Minuten auf 80°C erwärmt. Nach Abkühlung auf Raumtemperatur wurden 35 mg (0.91 mmol) Natriumborhydrid zugegeben. Man ließ 1 Stunde bei Raumtemperatur rühren, gab dann zur Reaktionsmischung Natriumhydrogencarbonat-Lösung und extrahierte mit Ethylacetat. Die organischen Phasen wurden eingeengt und der Rückstand chromatograhiert (Laufmittel:
Hexan/Ethylacetat/Methanol). Das so erhaltene Ethanolamin (223 mg) wurde zur Abspaltung der Benzylschutzgruppe in Methanol gelöst und mit 150 mg Palladiumhydroxid als Katalysator bei Raumtemperatur und Normaldruck hydriert. Der Katalysator wurde mittels Filtration über Celite® abgetrennt, das Filtrat vom Lösungsmittel befreit und der Rückstand chromatographiert (Kieselgel; Laufmittel: Dichlormethan/Methanol). Beigefarbener Feststoff. Ausbeute: 76 mg (22%); Massenspektrometrie: [M+H]⁺ = 375.

Die (R)- und (S)-Enantiomere dieses Ausführungsbeispiels können durch Auftrennung des Racemats in Analogie zu gängigen, im Stand der Technik bekannten Racematspaltungsmethoden erhalten werden.

In Analogie zu den vorstehend beschriebenen Synthesebeispielen können ferner die nachfolgenden Verbindungen der Formel 1 erhalten werden:
**Vergleichsbeispiel 15:** 8-{2-[2-(4-Fluor-3-methoxy-phenyl)-1,1-dimethyl-ethylamino]-1-hydroxyethyl}-6-hydroxy-4H-benzo [1,4]oxazin-3-on;
**Vergleichsbeispiel 16**: 8-{2-[2-(4-Fluor-2,6-dimethyl-phenyl)-1,1-dimethyl-ethylamino]-1-hydroxyethyl}-6-hydroxy-4H-benzo [1,4]oxazin-3-on;
**Vergleichsbeispiel 17**: 8- 2-{2-(4-Chlor-2-methyl-phenyl)-1,1-dimethyl-ethylamino]-1-hydroxyethyl}-6-hydroxy-4H-benzo [1,4]oxazin-3-on;
**Vergleichsbeispiel 18:** 8-{2-[2-(4-Chlor-3-fluor-phenyl)-1,1-dimethyl-ethylamino]-1-hydroxyethyl}-6-hydroxy-4H-benzo[1,4] oxazin-3-on;
**Vergleichsbeispiel 19**: 8-{2-[2-(4-Chlor-2-fluor-phenyl)-1,1-dimethyl-ethylamino]-1-hydroxyethyl}-6-hydroxy-4H-benzo[1,4] oxazin-3-on;
**Vergleichsbeispiel 20:** 8-{2-[2-(3-Chlor-4-fluor-phenyl)-1,1-dimethyl-ethylamino]-1-hydroxyethyl}-6-hydroxy-4H-benzo[1,4] oxazin-3-on;
**Vergleichsbeispiel 21:** 8-{2-[2-(2,6-Difluor-4-methoxy-phenyl)-1,1-dimethyl-ethylamino]-1-hydroxy-ethyl}-6-hydroxy-4H-benzo[1,4]oxazin-3-on;
**Vergleichsbeispiel 22:** 8-{2-[2-(2, 5-Difluor-4-methoxy-phenyl)-1,1-dimethyl-ethylamino]-1-hydroxy-ethyl}-6-hydroxy-4H-benzo[1,4]oxazin-3-on;
**Vergleichsbeispiel 23:** 8-{2-[2-[4-Fluor-3,5-dimethyl-phenyl)-1,1-dimethyl-ethylamino]-1-hydroxyethyl}-6-hydroxy-4H-benzo [1,4]oxazin-3-on;
**Vergleichsbeispiel 24**: 8- {2-[2-(3,5-Dichlor-phenyl)-1,1-dimethyl-ethylamino]-1-hydroxy-ethyl} -6-hydroxy-4H-benzo[1,4] oxazin-3-on;
**Vergleichsbeispiel 25**: 8-{2-[2-(4-Chlor-3-methyl-phenyl)-1,1-dimethyl-ethylamino]-1-hydroxyethyl}-6-hydroxy-4H-benzo[1,4] oxazin-3-on;
**Vergleichsbeispiel 26:** 8-{2-[2-(3,4,5-Trifluor-phenyl)-1,1-dimethyl-ethylamino]-1-hydroxy-ethyl}-6-hydroxy-4H-benzo[1,4] oxazin-3-on;
**Vergleichsbeispiel 27**: 8-{2-[2-(3-Methyl-phenyl)-1,1-dimethyl-ethylamino]-1-hydroxy-ethyl}-6-hydroxy-4H-benzo[1,4]oxazin-3-on;
**Vergleichsbeispiel 28**: 8-{2-[2-(3,4-Dichlor-phenyl)-1,1-dimethyl-ethylamino]-1-hydroxy-ethyl}-6-hydroxy-4H-benzo[1,4] oxazin-3-on.

Die Verbindungen derallgemeinen Formel 1 können allein oder in Kombination mit anderen erfindungsgemäßen Wirkstoffen der Formel 1 zur erfindungsgemäßen Anwendung gelangen. Gegebenenfalls können die Verbindungen der allgemeinen Formel 1 auch in Kombination mit weiteren pharmakologisch aktiven Wirkstoffen eingesetzt werden.

Es handelt sich hierbei insbesondere um Anticholinergika, gegebenenfalls andere Betamimetica, Antiallergika, PDE IV-Inhibitoren, PAF-Antagonisten, Leukotrien-Antagonisten und Corticosteroiden sowie Wirkstoffkombinationen davon.

Als bevorzugt Beispiele für Anticholinergika sind zu nennen Ipratropium-, Oxitropium-und Tiotropiumsalze. Arzneimittelkombinationen die neben den erfindungsgemäßen Verbindungen der Formel 1 vorstehend genannte Salze enthalten, enthalten bevorzugt solche Salze des Ipatropiums, Oxitropiums oder Tiotropiums in denen das Anion ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, gegebenenfalls in Form eines ihrer Solvate oder Hydrate.

Im Rahmen der vorliegenden Erfindung werden unter Corticosteroiden, die gegebenenfalls in Kombination mit der Verbindung der Formel 1 zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide und Dexametasone. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten, die gegebenenfalls in Kombination mit der Verbindung der Formen **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt Im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit der Verbindung der Formel 1 als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylllne, Roflumilast, Ariflo, Bay-198004, CP-325,366, BY343, D-4396 (Sch-351591), V-11294A und AWD-12-281. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-N-Inhibitoren gebildet werden können, werden erfindungsgemäß pharmazeutisch verträgliche Salze verstanden, die ausgewählt aus den Salzen derSaizsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. HilfLösungsmittel eingesetzt werden können, hergestellt und in Injektionsflaschen oder Ampullen oder Infusionsflaschen abgefüllt

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Fälle der oralen Anwendung können die Tabletten selbstverständlich aüßer den genannten Trägerstoffen auch Zusätze, wie z.B. Natriumcitrat, Calciumcarbonat und Dicalciumphosphatzusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäßen Applikation der Verbindungen der Formel **1** zur Therapie von COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.
Sind die Wirkstoffe 1 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zurAnwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhaltionspulverdurch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponentegelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oderalkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die 1 enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet. In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Tnhalationslösungen bevorzugt, in denen der Gehalt an Natriumedetat bei 0 bis 10mg/100ml liegt.
Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oderverlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/ 100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungs gemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im zug-bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen.

Ein weiterer Aspekt der vorliegenden Erfindung betrifft die vorstehend genannten pharmazeutischen Formulierungen, gekennzeichnet durch einen Gehalt einer Verbindung der Formel **1**, als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:

### Pharmazeutische Formulierungsbeispiele

A)

| Tabletten | pro Tablette |
|---|---|
| Wirkstoff **1** | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.
B)

| Tabletten | pro Tablette |
|---|---|
| Wirkstoff **1** | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.
C)

| Ampullenlösung | |
|---|---|
| Wirkstoff **1** | 50 mg. |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.
D)

| Dosieraerosol | |
|---|---|
| Wirkstoff **1** | 0,005 |
| Sorbitantrioleat | 0,1 |
| Monofluortrichlormethan und TG134a: TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).
E)

| Lösungen (in mg/100ml) | |
|---|---|
| Wirkstoff **1** | 333.3 mg |
| Benzalkoniumchlorid | 10.0 mg |
| EDTA | 50.0 mg |
| HCl (1n) | ad pH 3.4 |

Diese Lösung kann in üblicher Art und Weise hergestellt werden.
F)

| Inhalationpulver | |
|---|---|
| Wirkstoff **1** | 12 µg |
| Lactose Monohydrat | ad 25 mg |

Die Herstellung des Inhalationspulvers erfolgt in üblicher Art und Weise durch Mischen der einzelnen Bestandteile.

## Patentansprüche

1. Verbindung der Formel **1a**:

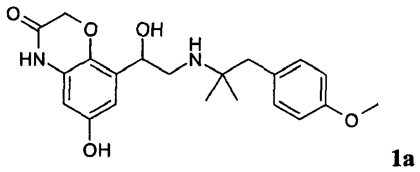

2. Verbindung der Formel **1b**:

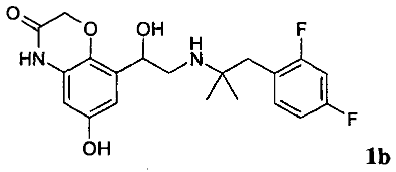

3. Verbindung der Formel **1c**:

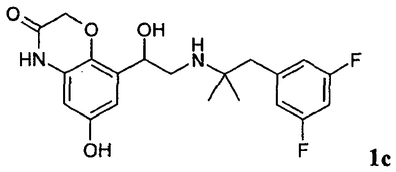

4. Verbindung der Formel **1d**:

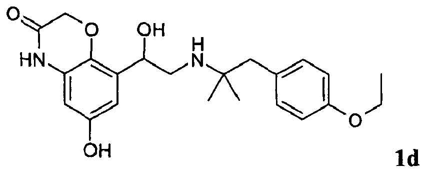

5. Verbindung der Formel **1e**:

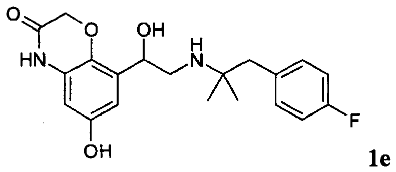

6. Verbindungen der Formeln **1a**, **1b**, **1c**, **1d** und **1e** nach einem der Ansprüche 1-5, worin die Verbindungen der Formeln **1a**, **1b**, **1c**, **1d** und le in Form der einzelnen optischen Isomeren, Mischungen der einzelnen Enantiomeren oder Racemate, bevorzugt in Form der enantiomerenreinen Verbindungen vorliegen.

7. Verbindungen der Formeln **1a**, **1b**, **1c**, **1d** und **1e** nach einem der Ansprüche 1-5, worin die Verbindungen der Formeln **1a**, **1b**, **1c**, **1d** und **1e** in Form ihrer Säureadditionssalze mit pharmakologisch unbedenklichen Säuren sowie gegebenenfalls in Form der Solvate und/oder Hydrate vorliegen.

8. Verbindungen der Formeln **1a**, **1b**, **1c**, **1d** und **1e** gemäß einem der Ansprüche 1-7 zur Verwendung als Arzneimittel.

9. Verbindungen der Formeln **1a**, **1b**, **1c**, **1d** und **1e** gemäß einem der Ansprüche 1-8 zur Verwendung als Arzneimittel zur Behandlung von COPD.

10. Pharmazeutische Formulierung, **gekennzeichnet durch** einen Gehalt einer Verbindung der Formel **1a**, **1b**, **1c**, **1d** oder 1**e** gemäß einem der Ansprüche 1 bis 7.

11. Inhalativ applizierbare pharmazeutische Formulierung, **gekennzeichnet durch** einen Gehalt einer Verbindung der Formel **1a**, **1b**, **1c**, **1d** oder 1**e** gemäß einem Ansprüche 1 bis 7.

12. Inhalativ applizierbare pharmazeutische Formulierung nach Anspruch 11, **dadurch gekennzeichnet, dass** sie ausgewählt ist aus der Gruppe bestehend aus Inhalationspulvern, treibgashaltigen Dosieraerosolen und treibgasfreien Inhalationslösungen.

## Claims

1. Compound of formula **1a**:

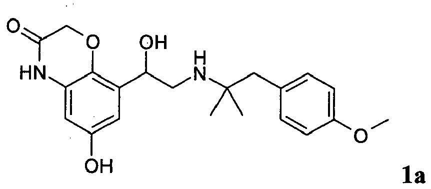

2. Compound of formula **1b**:

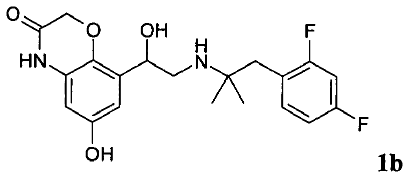

3. Compound of formula **1c**:

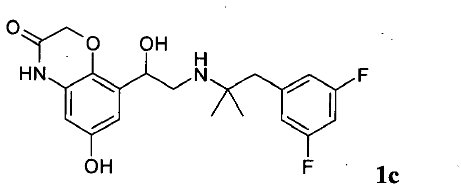

4. Compound of formula **1d**:

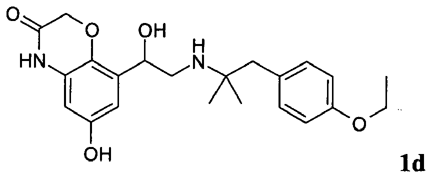

5. Compound of formula **1e**:

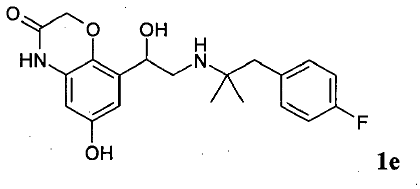

6. Compounds of formulas **1a**, **1b**, **1c**, **1d** and **1e** according to one of claims 1-5, wherein the compounds of formulas **1a**, **1b**, **1c**, **1d** and **1e** are present in the form of the individual optical isomers, mixtures of the individual enantiomers or racemates, preferably in the form of the enantiomerically pure compounds.

7. Compounds of formulas **1a**, **1b**, **1c**, **1d** and **1e** according to one of claims 1-5, wherein the compounds of formulas **1a**, **1b**, **1c**, **1d** and **1e** are present in the form of the acid addition salts thereof with pharmacologically acceptable acids as well as optionally in the form of the solvates and/or hydrates.

8. Compounds of formulas **1a**, **1b**, **1c**, **1d** and **1e** according to one of claims 1-7 for use as pharmaceutical compositions.

9. Compounds of formulas **1a**, **1b**, **1c**, **1d** and **1e** according to one of claims 1-8, for use as a pharmaceutical composition for the treatment of COPD.

10. Pharmaceutical formulation, **characterized in that** it contains a compound of formula **1a**, **1b**, **1c**, **1d** or **1e** according to one of claims 1 to 7.

11. Pharmaceutical formulation for administration by inhalation, **characterized in that** it contains a compound of formula **1a**, **1b**, **1c**, **1d** or **1e** according to one of claims 1 to 7.

12. Pharmaceutical formulation for administration by inhalation according to claim 11, **characterized in that** it is selected from among the inhalable powders, propellant-containing metered dose aerosols and propellant-free inhalable solutions.

## Revendications

1. Composé de formule **1a**:

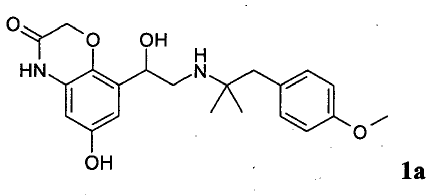

2. Composé de formule **1b**:

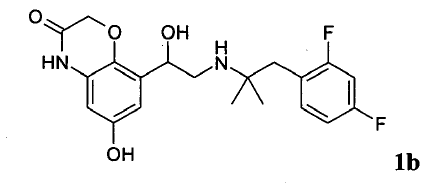

3. Composé de formule **1c**:

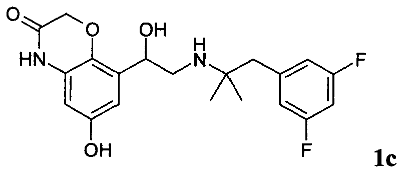

4. Composé de formule **1d**:

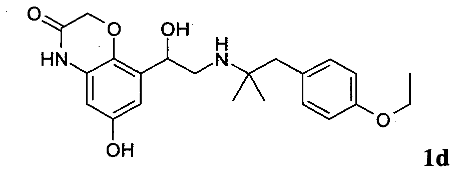

5. Composé de formule **1e**:

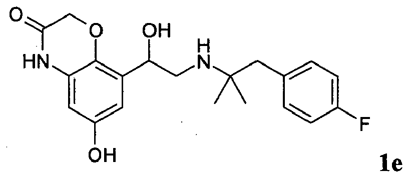

6. Composé de formule **1a**, **1b**, **1e**, **1d** et **1e** selon l'une des revendications 1 à 5 où les composés de formule **1a**, **1b**, **1e**, **1d** et **1e** sont sous forme des isomères optiques individuels, de mélanges d'énantiomères individuels ou de racémates, de préférence sous forme des composés énantiomériquement purs.

7. Composé de formule **1a**, **1b**, **1e**, **1d** et **1e** selon l'une des revendications 1 à 5 où les composés de formule **1a**, **1b**, **1e**, **1d** et **1e** sont sous forme de leurs sels d'addition d'acide avec des acides pharmacologiquement acceptables ainsi éventuellement que sous forme des solvates et/ou d'hydrates.

8. Composés de formule **1a**, **1b**, **1c**, **1d** et **1e** selon l'une des revendications 1 à 7 pour utilisation comme médicament.

9. Composés de formule **1a**, **1b**, **1c**, **1d** et **1e** selon l'une des revendications 1 à 8 pour utilisation comme médicament pour le traitement de la COPD.

10. Formulation pharmaceutique **caractérisée par** une teneur en un compose de formula **1a**, **1b**, **1c**, **1d** ou **1e** selon l'une des revendications 1 à 7.

11. Formulation pharmaceutique applicable par inhalation **caractérisée par** une teneur en un composé dé formule **1a**, **1b**, **1c**, **1d** ou **1e** selon l'une des revendications 1 à 7.

12. Formulation pharmaceutique applicable par inhalation selon la revendication 11 **caractérisée en ce qu'**elle est choisie dans le groupe consistant en les poudres pour inhalation, les aérosols doseurs contenant un gaz propulseur et les solutions pour inhalation sans gaz propulseur.
